# EUROPEAN PATENT APPLICATION

(11) **EP 4 427 673 A1**
(43) Date of publication of application: **11.09.2024**
(21) Application number: 22889918.3
(22) Date of filing: 28.10.2022
(51) Int. Cl.: A61B 5/11, A61B 5/00, A61B 5/026, A61B 5/16, A61B 7/04

(54) **HEAD BIOLOGICAL SIGNAL DETECTION DEVICE AND BIOLOGICAL STATE ESTIMATION DEVICE**

(30) Priority: 04.11.2021 JP 2021180168
(71) Applicant: Delta Tooling Co., Ltd., Hiroshima-shi, Hiroshima 736-0084 (JP)
(72) Inventor: FUJITA, Etsunori, Hiroshima-shi, Hiroshima 736-0084 (JP); OGURA, Yumi, Hiroshima-shi, Hiroshima 736-0084 (JP); UCHIKAWA, Ryuichi, Hiroshima-shi, Hiroshima 736-0084 (JP); BABA, Daichi, Hiroshima-shi, Hiroshima 736-0084 (JP)
(74) Representative: Hoffmann Eitle
(86) International application number: PCT/JP2022/040550
(87) International publication number: WO 2023/080098

(57) **Abstract**

With a simple structure, to make it possible to detect a head biological signal that reflects the state of cerebral blood flow, and to make it possible to assess a sleep stage based on a variation in the cerebral blood flow. A head biological signal detection sensor (10) is supported on the head using a sensor support member (20) such that a connecting yarn sags to make an interval between a pair of facing ground knitted fabrics in a three-dimensional knitted fabric (11) narrower than that under no load. With this configuration, microvibration occurring in yarns and fibers forming the three-dimensional knitted fabric of the head biological signal detection sensor or in other places due to head movement resulting from body motion or the like generates stochastic resonance, which makes it possible to detect a head biological signal.

## Description

### Technical Field

The present invention relates to a head biological signal detection device that detects a head biological signal and a biological state estimation device that estimates the state of a person using the detected head biological signal.

### Background Art

In Patent Documents 1 to 4, the present inventors have proposed an art to capture, in a non-constraining manner, vibration generated on the dorsal body surface of a person and estimate the state of the person by analyzing the vibration. The vibration generated on the dorsal body surface of a person is vibration propagated from a human body's inner part such as the heart and the aorta and contains information on atrial and ventricular systoles and diastoles, information on vascular wall elasticity which serves as an auxiliary pump for circulation, and information on reflected waves.

In Patent Document 1, slide calculation is performed in which a predetermined time width is set in a time-series waveform of a dorsal body surface pulse wave of around 1 Hz extracted from vibration (biological signal) propagated through the body surface to find a frequency slope time-series waveform, and from the tendency of its variation, for example, based on whether its amplitude is on the increase or on the decrease, a biological state is estimated. It is also disclosed that power spectra of frequencies respectively corresponding to a function regulation signal, a fatigue reception signal, and an activity regulation signal that belong to predetermined ranges from the ULF band (ultra-low-frequency band) to the VLF band (very-low-frequency band) are found through a frequency analysis of biological signals, and the state of a person is determined from time-series variations of the respective power spectra.

Patent Documents 2, 3 disclose a means for determining a homeostasis function level. Further, Patent Document 4 discloses a sound/vibration information collection mechanism including a resonance layer provided with a natural oscillator having a natural frequency corresponding to sound/vibration information of a biological signal.

### Prior Art Document

### Patent Document

Patent Document 1: Japanese Patent Application Laid-open No. 2011-167362
Patent Document 2: Japanese Patent Application Laid-open No. 2014-117425
Patent Document 3: Japanese Patent Application Laid-open No. 2014-223271
Patent Document 4: Japanese Patent Application Laid-open No. 2016-26516

### Summary of the Invention

### Problems to Be Solved by the Invention

The sensor that captures, in a non-constraining manner, the vibration generated on the body surface as is disclosed in Patent Documents 1 to 4 is composed of a three-dimensional knitted fabric, a film surrounding the three-dimensional knitted fabric, a microphone, and so on, and is capable of capturing biological signals through the body surface only by being in contact with the person. That is, it is possible to obtain biological signal data only by attaching the device to the body of a person, without any operation by a doctor or the like.

Abnormal cerebral blood flow, brain metabolism, and cerebrospinal fluid seriously affect a brain function. The complete stop of cerebral blood flow results in no oxygen supply and accordingly, the stop of the metabolism of brain cells, leading to a loss of consciousness five to ten seconds later.

Cerebral blood flow is supplied through the four major arteries, that is, the two carotid arteries and the two vertebral arteries. In the head, these arteries connect to the anterior cerebral arteries, the middle cerebral arteries, the posterior cerebral arteries, and the anterior and posterior communicating arteries to form the arterial circle of Willis at the base of the brain. Further, the anterior cerebral arteries, the middle cerebral arteries, and the posterior cerebral arteries branch out into the pial arteries, which run on the brain surface. Therefore, arterial sound starting from the heart is transmitted to a connection part between the spinal column and the cranium and is further transmitted from the pial arteries running on the brain surface to the cranium. Therefore, there is a possibility that sound of these arteries can be captured through the cranium.

The autoregulation of cerebral blood flow works when a blood pressure is within a range of 60 to 140 mmHg, but when the blood pressure decreases to 60 mmHg or lower, cerebral blood flow rapidly reduces. Further, the arteries on the brain surface are governed by nociceptive C fibers, parasympathetic vasodilator nerves, and sympathetic vasoconstrictor nerves, and for example, vasodilation due to the C fibers is involved in hyperemia occurring in a headache stage of meningitis, epilepsy, or migraine.

Even a change in the environmental condition does not cause a great variation in the rate of cerebral blood flow in cerebral circulation as long as the arterial blood pressure is kept at a certain level or higher, but the distribution of blood flow in the brain greatly varies depending on the brain activity, and a large amount of blood is sent to a cortex region where the activity is high.

Owing to the function of autoragulating the cerebral blood flow, its communication with the internal carotid arteries and the vertebral arteries in the brainstem occurs, ensuring the safety of cerebral perfusion. For example, even if the internal carotid artery on one side is partly closed, the blood flow in the other communicating artery increases to improve the cerebral perfusion in the affected part. Cerebral blood flow is determined by a perfusion pressure, which is a difference between an arterial pressure and a venous pressure, and by vascular resistance. Cerebral circulation is in the hard skull and is greatly influenced by a change in ICP (Intracranial pressure). ICP is a pressure in the space present between the brain parenchyma and the skull and filled with liquid. For example, if a cerebral hemorrhage or a cerebral edema occurs due to an external head injury or if a brain tumor becomes larger, ICP increases. The increase in ICP results in vascular compression to reduce cerebral blood flow. The vascular compression reduces a damping ratio and increases vascular elasticity, generating a damped free vibration waveform having only low-frequency components with low damping. An increase in blood flow increases a damping ratio and reduces vascular elasticity, generating a damped free vibration waveform with high damping in which harmonic components with high damping and small amplitudes are superimposed and thus low-frequency and high-frequency components are mixed. ICP is typically higher than an extracranial venous pressure, and an effective cerebral perfusion pressure is a value equal to an average arterial pressure from which ICP is subtracted. In the case where ICP increases and a systemic blood pressure is low, a cerebral perfusion pressure and cerebral blood flow reduce. On the other hand, if the parasympathetic function becomes active in a drowsy state to cause peripheral vasodilation, cerebral blood flow increases.

Further, during sleeping and while a wakefulness degree is low, blood supply to the brainstem playing the role of controlling the movement and balance of the whole body varies only a little, but blood supply to the motor cortex greatly reduces.

Further, the cerebellum is considered to play the role of a typical feedback control circuit in the balance control of the body to be responsible for the function of predictively correcting the posture using information from the peripheries of the body and the vestibular organ, and it plays the role of maintaining body balance at the time of a very quick movement such as a sudden change of a motion direction. Then, the cerebellum works as a damping system regarding motion control by the nervous system to damp the motion. Therefore, the cerebellum is involved in muscle controls at all levels. Further, neurotransmitters in the basal ganglia are involved in behavioral, sleep-wake, and autonomic nervous system functions.

In a sleepy state, these functions are impaired, which causes a blood supply variation accompanying sleepiness. By thus capturing a variation in cerebral blood flow, it is possible to assess a state associated with sleep.

Meanwhile, the international criteria for human sleep stage determination classify sleep into a wake stage (Stage W), a drowsiness stage + a very light sleep stage = Stage 1, a light sleep stage (Stage 2), a moderately deep sleep stage (Stage 3), a deep sleep stage (Stage 4), and a REM sleep stage (Stage REM). The assessment of these is conducted in association with brain waves, and the assessment of a sleep stage using biological signals other than brain waves is not usually done. Further, an electroencephalograph amplifies weak electrical activities generated by the brain to record them, and because of a need to attach its electrodes to the head, its measurement in home and offices is difficult and is conducted in specialized examination institutions such as medical institutions.

Brain waves are thus typically used for the assessment of a sleep stage, but a variation in cerebral blood flow is not used for the assessment of sleep though directly correlated with a brain state change as described above. Since sleep is highly correlated with brain neural activity, it is thought that capturing, as a blood flow variation, vibration propagated to the head surface through the skull enables relatively higher-accuracy capturing of a brain state change than capturing data measured at the thorax or the back. Means for measuring cerebral blood flow include an N₂O method (average blood flow), a thermocouple method (qualitative), a microsphere method (quantitative local blood flow measurement), a radioautography method (quantitative local blood flow measurement), and a hydrogen clearance method, but they are conducted only in medical institutions or specialized examination institutions, and it is not easy to capture a variation in cerebral blood flow.

The present invention was made in consideration of the above and has an object to provide a head biological signal detection device and a biological state estimation device that have simple structures yet enable the capturing of a head biological signal reflecting the state of cerebral blood flow, thereby enabling the assessment of a sleep stage based on a variation in the cerebral blood flow, and that further enable the estimation of a change in body state other than the sleep stage through the analysis of the head biological signal.

### Means for Solving the Problems

To solve the aforesaid problems, the present inventors first considered applying the biological signal detection device including the three-dimensional knitted fabric, the microphone, and so on, which is disclosed in the aforesaid Patent Documents 1 to 4. However, those disclosed in Patent Documents 1 to 4 all are attached to the trunk, in particular, the back, and at the body surface to which cardiac and aortic motions are relatively easily transmitted, capture their sound/vibration. Of course, this sound/vibration at the body surface of the trunk is also weak, and it is sure that those in Patent Documents 1 to 4 are excellent in capturing such weak signals, but sound/vibration at the head is weaker as a signal transmitted to the head surface than that transmitted to the body surface of the trunk because the head is surrounded by the skull. This makes it difficult to capture the biological signal at the head only by using the structure of Patent Documents 1 to 4 as it is. Here, the present inventors noticed that the use of the sagging of a connecting yarn forming the three-dimensional knitted fabric increases detection sensitivity owing to stochastic resonance, and thought that this would enable the capturing of a head biological signal and has completed the present invention.

Further, the present inventor analyzed head biological signals captured in this manner and has found that a variation tendency of head biological signals with the same frequencies as the frequencies of brain waves used in the assessment of a sleep stage is similar to a tendency presented when the brain wave frequency varies according to a sleep stage.

Specifically, a head biological signal detection device of the present invention includes:
a head biological signal detection sensor that includes: a three-dimensional knitted fabric having a pair of ground knitted fabrics facing each other at an interval and a connecting yarn knitted between the pair of ground knitted fabrics to keep the interval between the pair of facing ground knitted fabrics; a housing film covering the three-dimensional knitted fabric; and a microphone that detects sound/vibration propagated through the three-dimensional knitted fabric, the head biological signal detection sensor being disposed with an abutting surface thereof in contact with a surface of a head, to detect a head biological signal which is sound/vibration generated by cerebral blood flow; and
a sensor support member with which the head biological signal detection sensor is supported on the head in a manner that the abutting surface is in contact with the surface of the head and the connecting yarn sag to make the interval between the pair of facing ground knitted fabrics narrower than that under no load,
wherein microvibration occurring in the three-dimensional knitted fabric or the housing film due to movement of the head while the biological signal detection device is worn works as noise to generate a stochastic resonance phenomenon between the noise and the head biological signal, and the head biological signal is detected by the microphone.

Preferably, the sensor support member has a worn state maintaining structure that, when the head biological signal detection sensor is supported on the head, causes the connecting yarn to sag such that the interval between the pair of facing ground knitted fabrics becomes a 40 to 95% interval of a reference interval under no load.

Preferably, the sensor support member is of a cap type, on an inner peripheral surface of a crown of the sensor support member, the head biological signal detection sensor is attached and on a lower edge of the crown, a fastening structure as the worn state maintaining structure is provided to maintain a position of the head biological signal detection sensor by being in close contact with a periphery of the head.

Preferably, the fastening structure includes at least one of an elastic member exhibiting an elastic force in a direction of the periphery of the head and a belt-shaped member whose length is adjustable.

Preferably, the crown is formed of a three-dimensional knitted fabric.

Preferably, the microphone of the head biological signal detection sensor is attached to an outer side of the housing film,
the head biological signal detection sensor has a synthetic resin case covering the microphone and an external disturbance mixture preventing member functioning in the case to prevent an external disturbance from mixing to the microphone, and
a surface, of the housing film, opposite to the surface to which the microphone is attached is the abutting surface.

Preferably, the external disturbance mixture preventing member is a gel.

Further, a biological state estimation device of the present invention includes a head biological signal analysis unit that receives and analyzes the head biological signal obtained from the above-described head biological signal detection device, estimates a predetermined state of a person together with an occurrence instant of the state, and outputs a result of the estimation.

Preferably, the head biological signal analysis unit includes a brain activity state analysis unit that outputs an index regarding an activity state of a brain, together with an occurrence instant of the index.

Preferably, the brain activity state analysis unit includes a sleep stage identifying unit that frequency-analyzes the head biological signal obtained from the head biological signal detection device to find, in time series, a main frequency band of the head biological signal, and based on the main frequency band, identifies a sleep stage out of sleep stages including a wake stage, a drowsiness stage to a deep sleep stage, and a REM sleep stage.

Preferably, the brain activity state analysis unit includes a wakefulness degree change index estimation unit that, in a time-series waveform of the head biological signal obtained from the head biological signal detection device, identifies a waveform component whose amplitude is larger than preceding and succeeding amplitudes by a predetermined magnitude or more, and estimates that the waveform component is an index indicating a change in a wakefulness degree.

Preferably, the head biological signal analysis unit includes a heartbeat-related biological signal analysis unit that extracts, from the time-series waveform of the head biological signal obtained from the head biological signal detection device, a heartbeat-related biological signal generated by cardiac motion and containing at least one of an apical beat component and a heart sound component, and analyzes the heartbeat-related biological signal.

Preferably, the sleep stage identifying unit compares an instant when the amplitude of the time-series waveform obtained from the head biological signal detection device becomes large with a respiratory period, and identifies the sleep stage based on whether or not the instant coincides with the respiratory period.

### Effect of the Invention

The head biological signal detection device of the present invention is configured such that, with the sensor support member, the head biological signal detection sensor is supported on the head in the manner that the connecting yarn sags to make the interval between the facing ground knitted fabrics of the three-dimensional knitted fabric narrower than the interval under no load. Owing to the work of elasticity (reaction force) caused by the sagging of the connecting yarn, in yarns and fibers forming the three-dimensional knitted fabric of the head biological signal detection sensor and the housing film covering the three-dimensional knitted fabric, the microvibration accompanying the head movement resulting from the body motion or the like more easily occurs than in the case where the head biological signal detection device is brought into contact with the head under no load without any sagging of the connecting yarn. As a result, the stochastic resonance is more easily generated to increase detection sensitivity, making it possible to detect the head biological signal.

The head biological signal is sound/vibration generated by cerebral blood flow that is transmitted to the brain from the heart through the internal carotid arteries and the external carotid arteries, and by analyzing the head biological signal, it is possible to estimate the state of the cerebral blood flow and a body state change caused by the cerebral blood flow. In particular, in the present invention, the correlation between the time-series waveform of the head biological signal and the sleep stage is newly found, and consequently, it becomes possible to assess the sleep stage by capturing the head biological signal. Therefore, the use of the head biological signal detection device of the present invention makes it possible to daily estimate a sleep stage or capture a health condition involved in cerebral blood flow in homes, offices, and so on, as a pre-stage of close examination in medical institutions or the like.

### Brief Description of Drawings

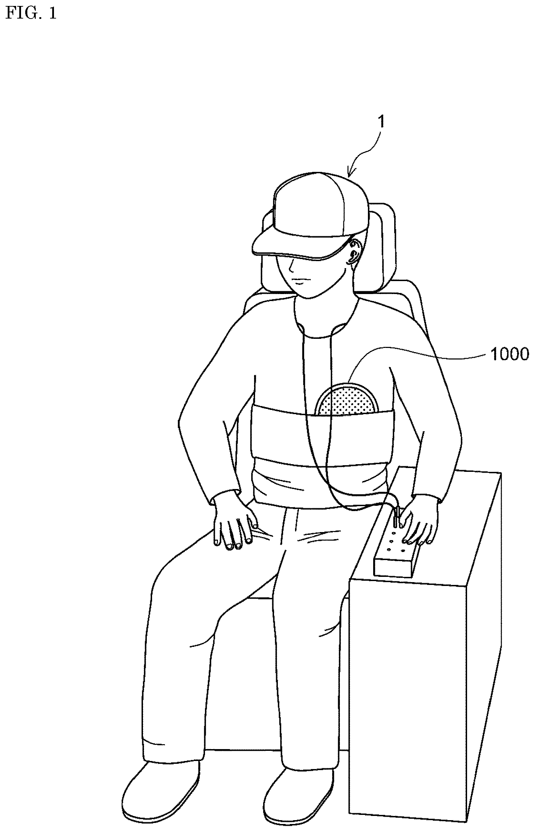

[FIG. 1] FIG. 1 is a view illustrating a state in which a head biological signal detection device of the present invention is worn on a subject.

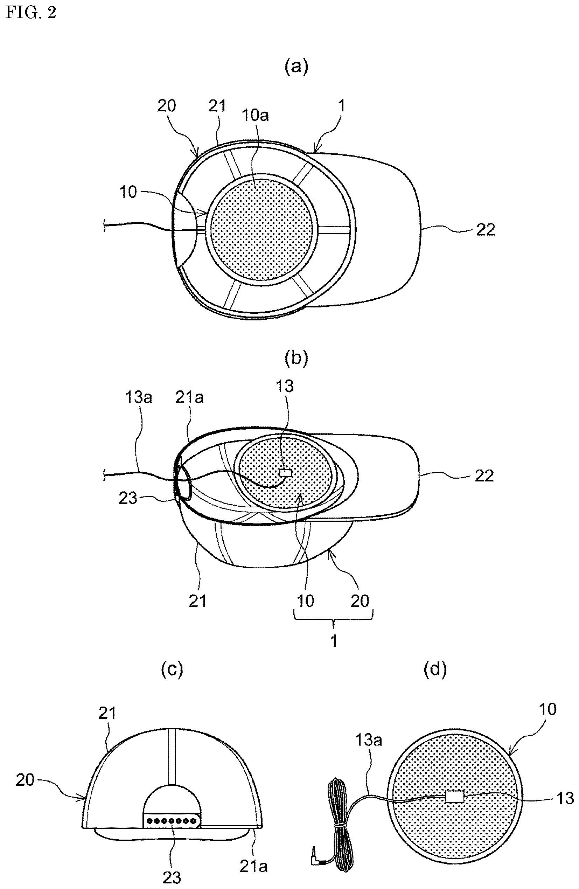

[FIGs. 2] FIG. 2(a) is a view of the head biological signal detection device seen from a lower edge side of a crown of a cap-type sensor support member, FIG. 2(b) is a view where a head biological signal detection sensor is detached from the vicinity of the top of the crown of the cap-type sensor support member and a surface where a microphone is attached is turned toward the lower edge of the crown, FIG. 2(c) is an explanatory view of a fastening structure provided on the cap-type sensor support member, and FIG. 2(d) is a view of the head biological signal detection sensor seen from the surface where the microphone is attached.

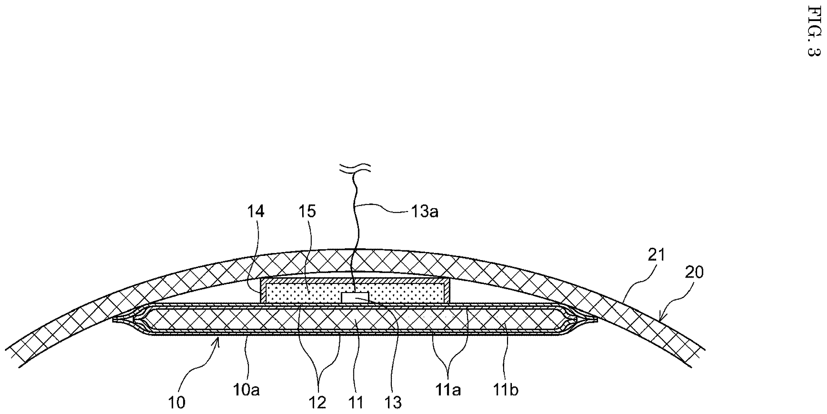

[FIG. 3] FIG. 3 is a sectional view of the head biological signal detection sensor and the cap-type sensor support member to explain their arrangement positions and configurations.

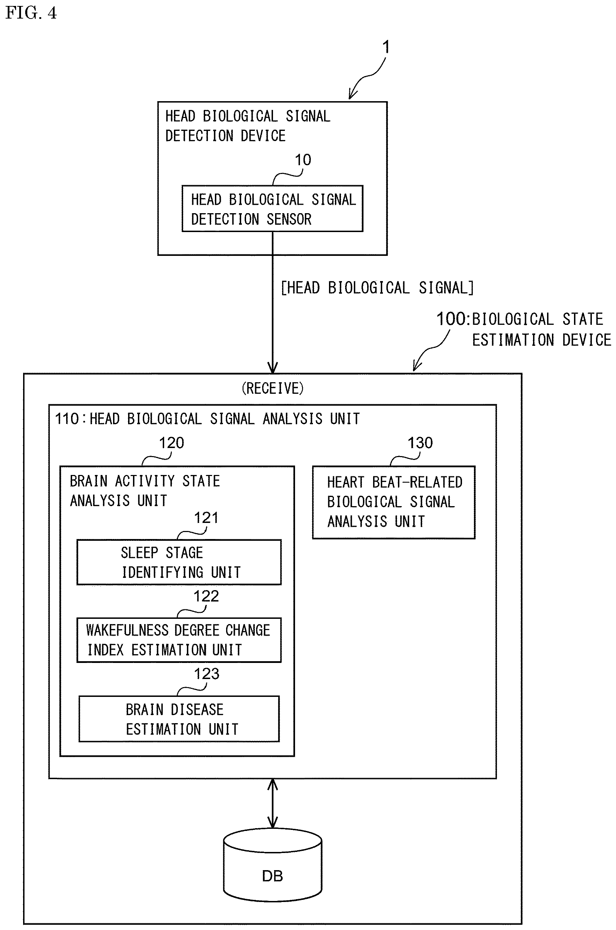

[FIG. 4] FIG. 4 is a diagram schematically illustrating the configuration of a biological state estimation device.

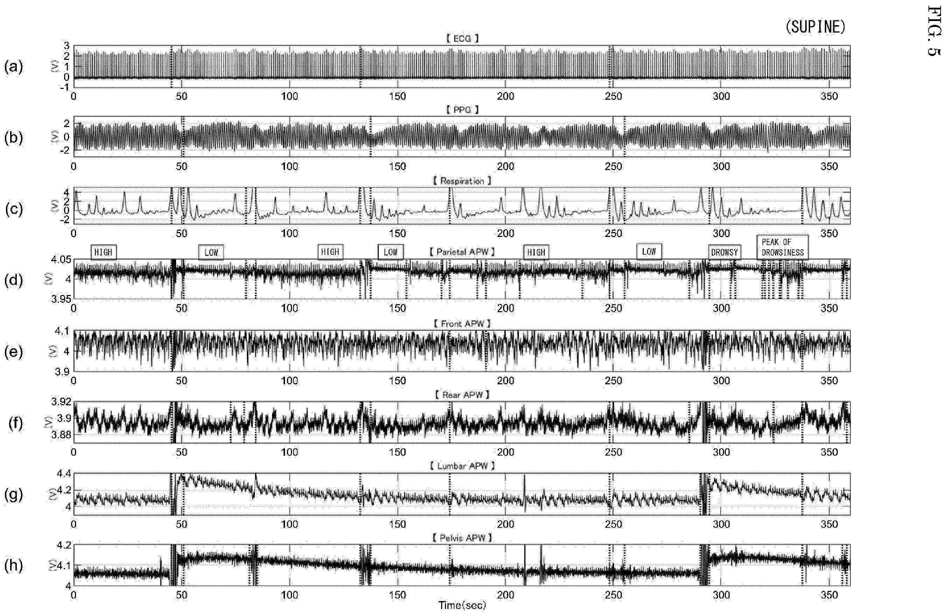

[FIGs. 5] FIGs. 5 are charts illustrating the results of a sleep experiment conducted while the subject is supine, (a) being a waveform of an electrocardiogram, (b) being a waveform of a finger plethysmogram, (c) being a respiratory waveform, (d) being a time-series waveform of a head biological signal, (e) being a time-series waveform of a thorax biological signal, (f) illustrating a time-series waveform of a dorsal biological signal, (g) being a time-series waveform of a lumbar biological signal, and (h) being a time-series waveform of a buttocks biological signal.

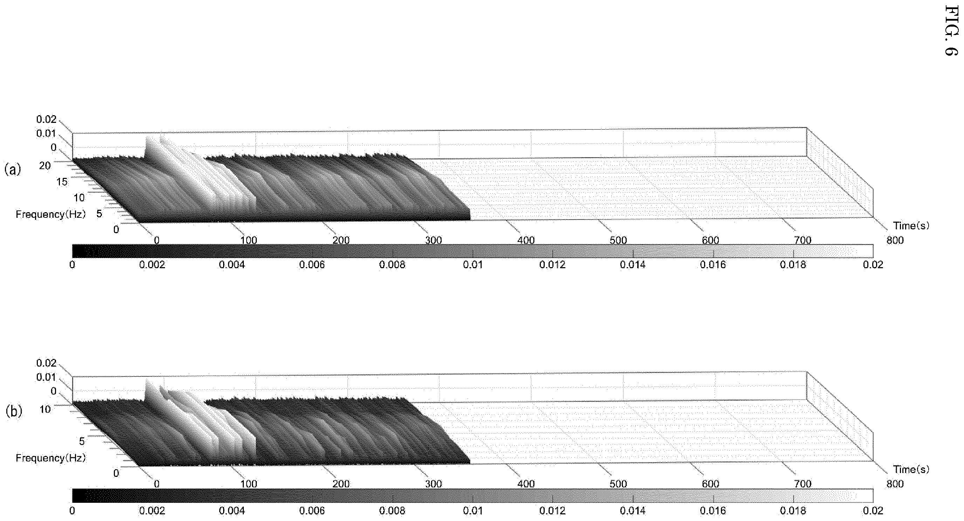

[FIGs. 6] FIGs. 6 are charts illustrating the results of a sleep experiment 1 conducted while a subject is supine, (a) being a chart illustrating, in time series, the result of a frequency analysis in a frequency band of 0 to 20 Hz, and (b) being a chart illustrating, in time series, the result of a frequency analysis in a frequency band of 0 to 10 Hz.

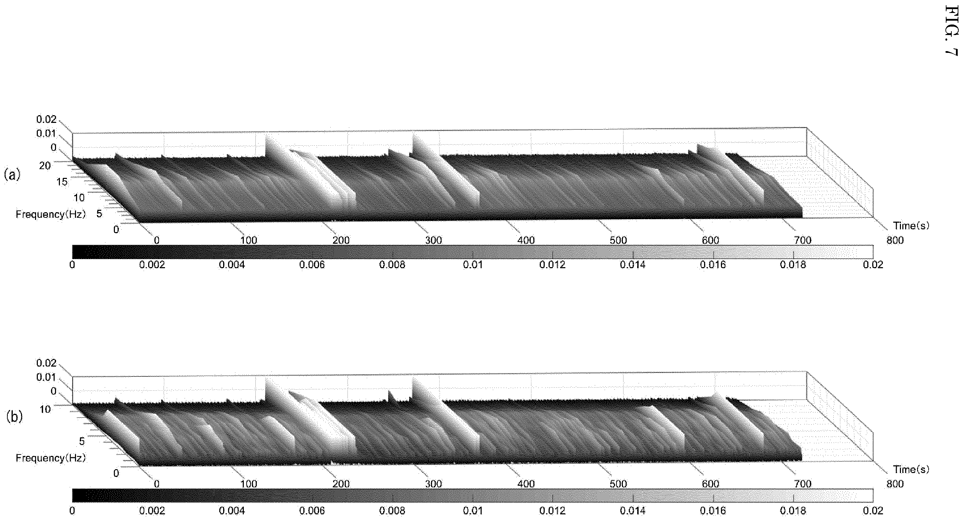

[FIGs. 7] FIGs. 7 are charts illustrating the results of the sleep experiment 1 conducted while the subject is left lateral recumbent, (a) being a chart illustrating, in time series, the result of a frequency analysis in a frequency band of 0 to 20 Hz, and (b) being a chart illustrating, in time series, the result of a frequency analysis in a frequency band of 0 to 10 Hz.

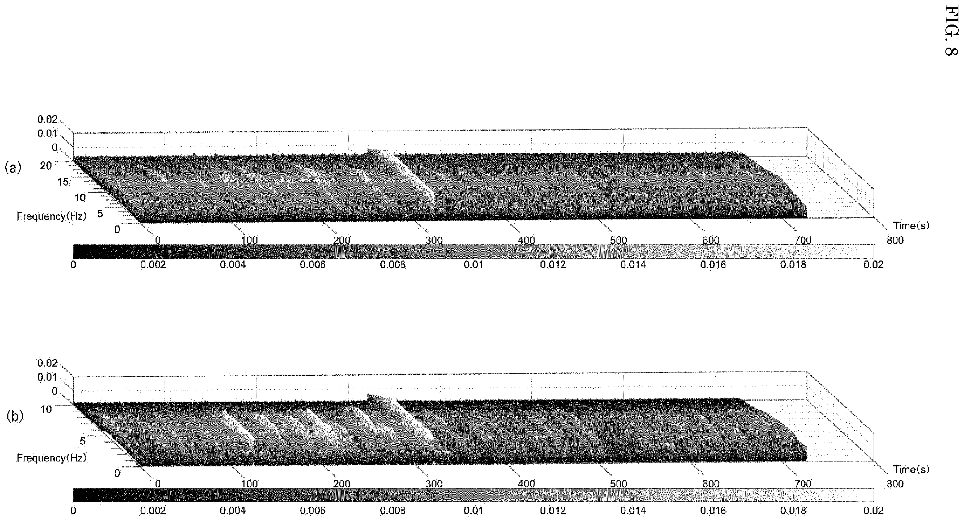

[FIGs. 8] FIGs. 8 are charts illustrating the results of the sleep experiment 1 conducted while the subject is right lateral recumbent, (a) being a chart illustrating, in time series, the result of a frequency analysis in a frequency band of 0 to 20 Hz, and (b) being a chart illustrating, in time series, the result of a frequency analysis in a frequency band of 0 to 10 Hz.

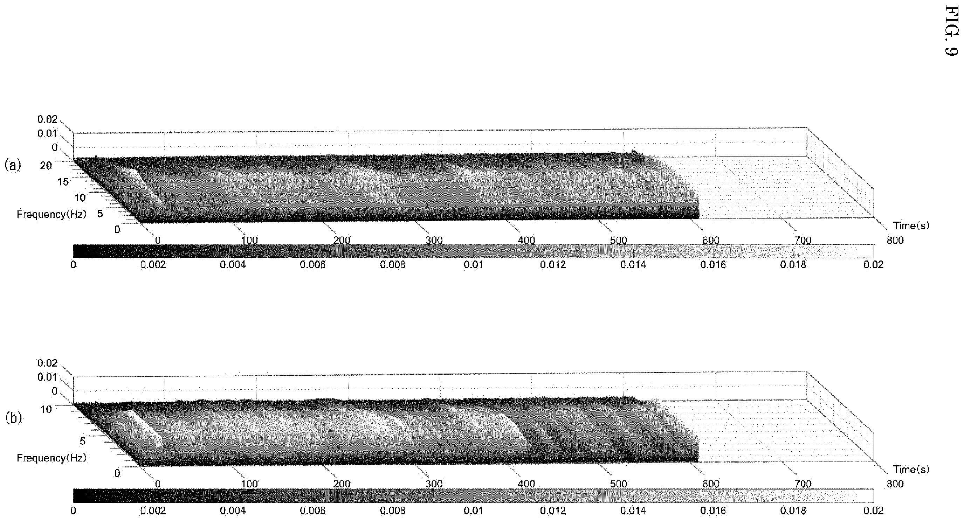

[FIGs. 9] FIGs. 9 are charts illustrating the results of the sleep experiment 1 while the subject is sitting, (a) being a chart illustrating, in time series, the result of a frequency analysis in a frequency band of 0 to 20 Hz, and (b) being a chart illustrating, in time series, the result of a frequency analysis in a frequency band of 0 to 10 Hz.

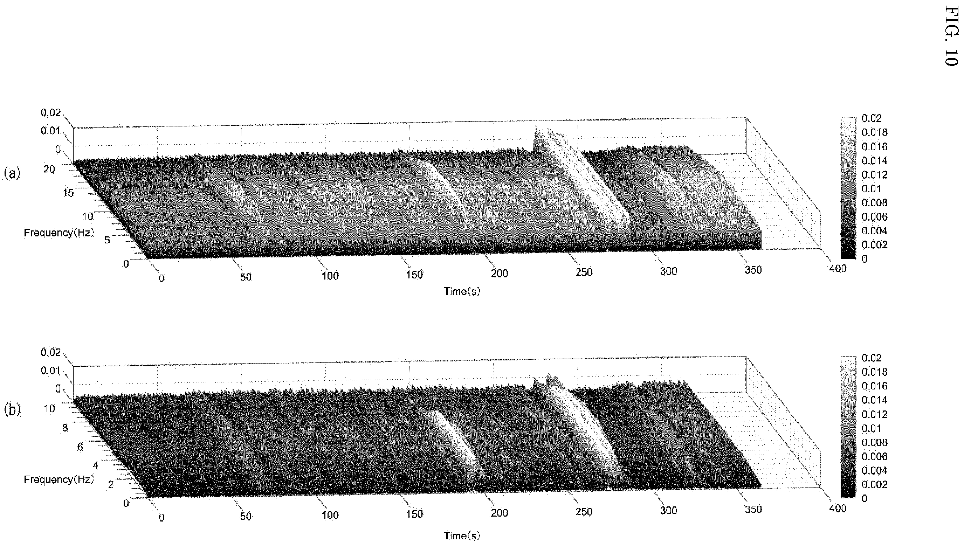

[FIGs. 10] FIGs. 10 are charts illustrating the results of a sleep experiment 2 conducted while the subject is supine, (a) being a chart illustrating, in time series, the result of a frequency analysis in a frequency band of 0 to 20 Hz, and (b) being a chart illustrating, in time series, the result of a frequency analysis in a frequency band of 0 to 10 Hz.

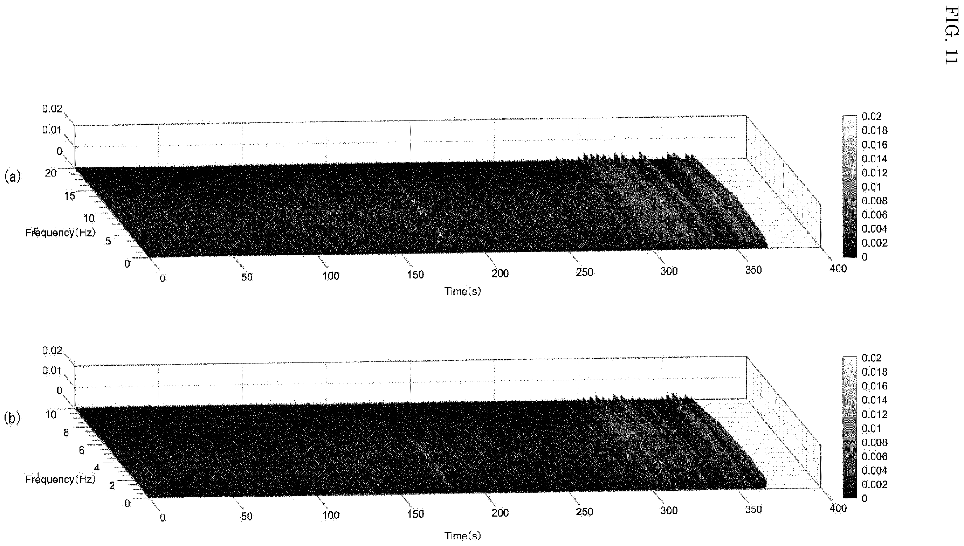

[FIGs. 11] FIGs. 11 are charts illustrating the results of the sleep experiment 2 conducted while the subject is left lateral recumbent, (a) being a chart illustrating, in time series, the result of a frequency analysis in a frequency band of 0 to 20 Hz, and (b) being a chart illustrating, in time series, the result of a frequency analysis in a frequency band of 0 to 10 Hz.

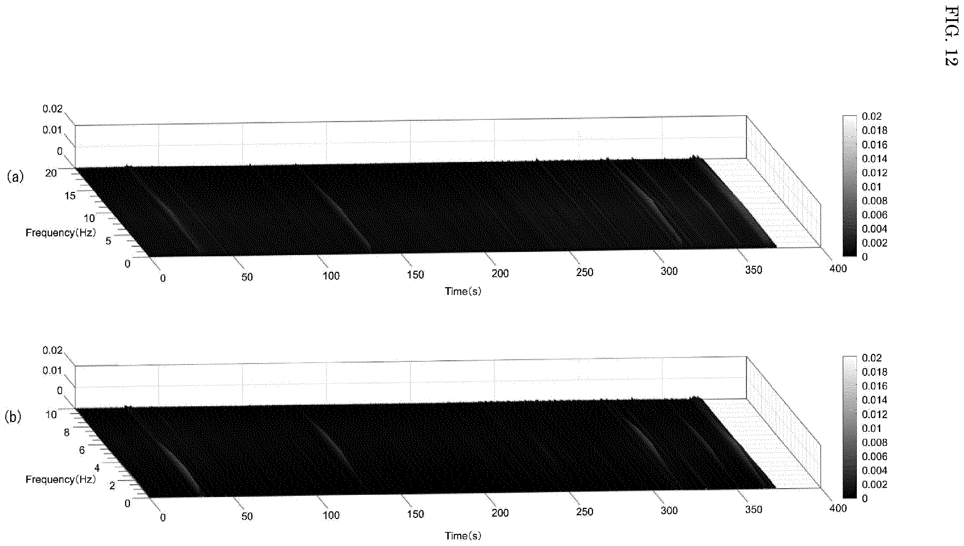

[FIGs. 12] FIGs. 12 are charts illustrating the results of the sleep experiment 2 conducted while the subject is right lateral recumbent, (a) being a chart illustrating, in time series, the result of a frequency analysis in a frequency band of 0 to 20 Hz, and (b) being a chart illustrating, in time series, the result of a frequency analysis in a frequency band of 0 to 10 Hz.

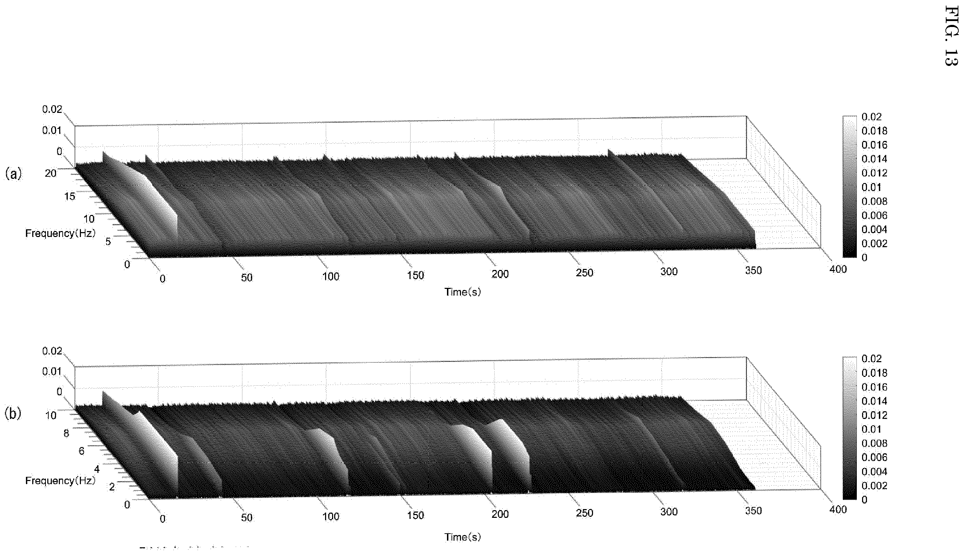

[FIGs. 13] FIGs. 13 are charts illustrating the results of the sleep experiment 2 conducted while the subject is sitting, (a) being a chart illustrating, in time series, the result of a frequency analysis in a frequency band of 0 to 20 Hz, and (b) being a chart illustrating, in time series, the result of a frequency analysis in a frequency band of 0 to 10 Hz.

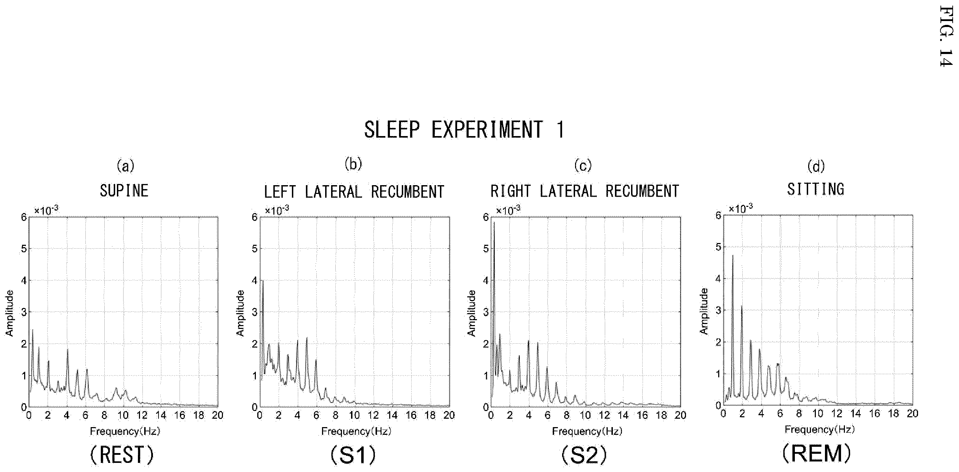

[FIGs. 14] FIGs. 14(a) to (d) are charts illustrating the frequency analysis results in the sleep experiment 1 conducted while the subject is in the respective postures.

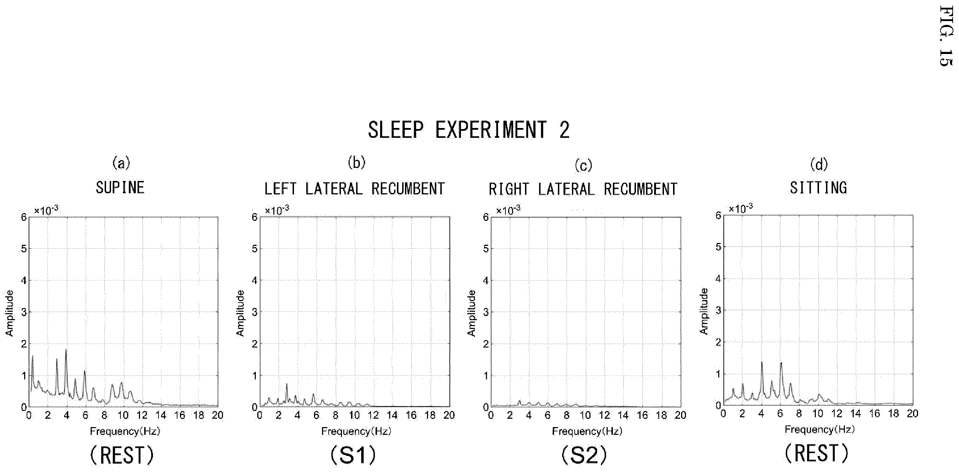

[FIGs. 15] FIGs. 15(a) to (d) are charts illustrating the frequency analysis results in the sleep experiment 2 conducted while the subject is in the respective postures.

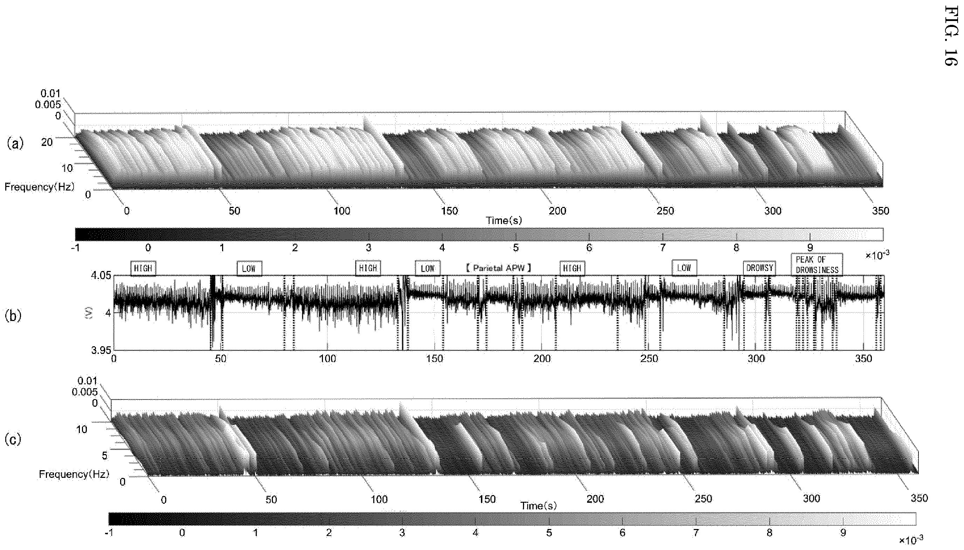

[FIGs. 16] FIGs. 16 are charts illustrating the results of a sleep experiment 3 conducted while the subject is supine, (a) being a chart illustrating, in time series, the result of a frequency analysis in a frequency band of 0 to 20 Hz regarding a head biological signal illustrated in (b), (b) being a time-series waveform of the head biological signal, and (c) being a chart illustrating, in time series, the result of a frequency analysis in a frequency band of 0 to 10 Hz regarding the head biological signal illustrated in (b).

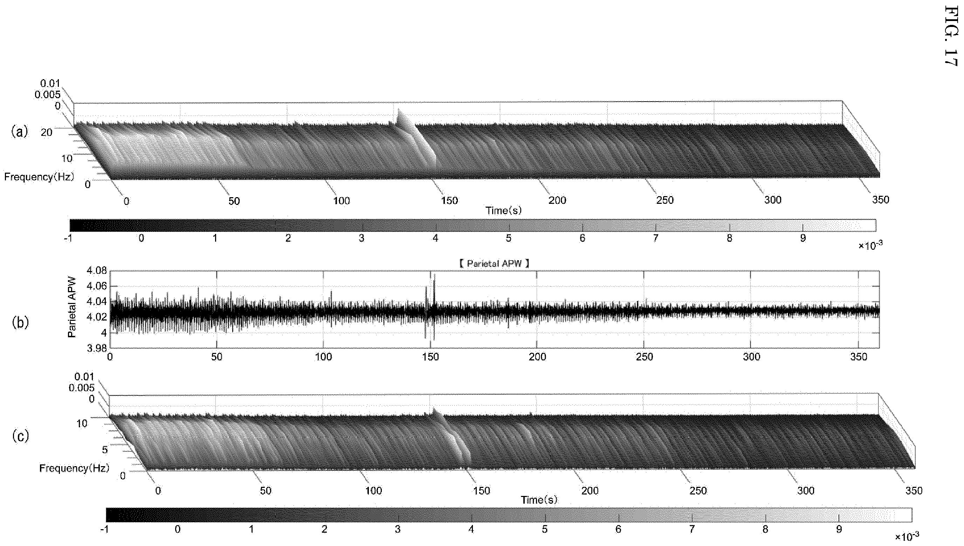

[FIGs. 17] FIGs. 17 are charts illustrating the results of the sleep experiment 3 conducted while the subject is left lateral recumbent, (a) being a chart illustrating, in time series, the result of a frequency analysis in a frequency band of 0 to 20 Hz regarding a head biological signal illustrated in (b), (b) being a time-series waveform of the head biological signal, and (c) being a chart illustrating, in time series, the result of a frequency analysis in a frequency band of 0 to 10 Hz regarding the head biological signal illustrated in (b).

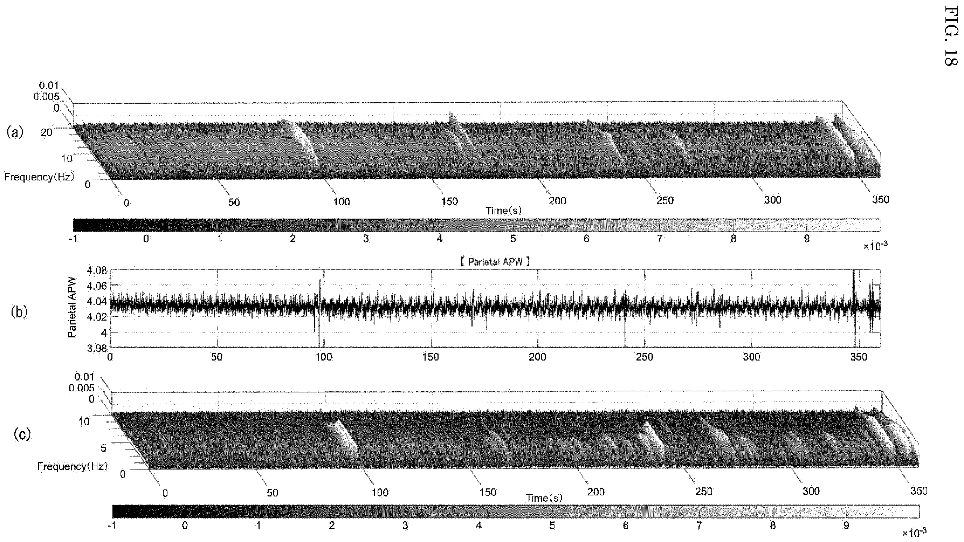

[FIGs. 18] FIGs. 18 are charts illustrating the results of the sleep experiment 3 conducted while the subject is right lateral recumbent, (a) being a chart illustrating, in time series, the result of a frequency analysis in a frequency band of 0 to 20 Hz regarding a head biological signal illustrated in (b), (b) being a time-series waveform of the head biological signal, and (c) being a chart illustrating, in time series, the result of a frequency analysis in a frequency band of 0 to 10 Hz regarding the head biological signal illustrated in (b).

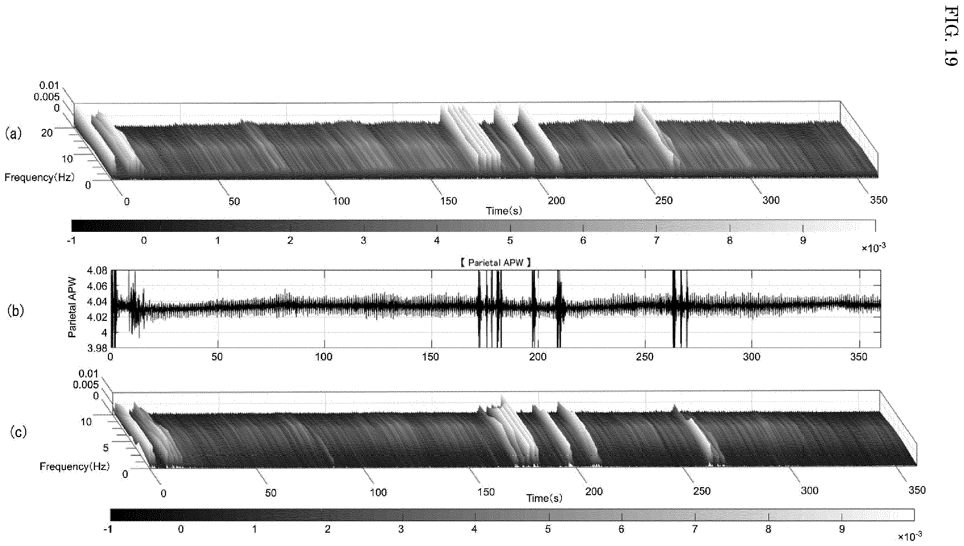

[FIGs. 19] FIGs. 19 are charts illustrating the results of the sleep experiment 3 conducted while the subject is sitting, (a) being a chart illustrating, in time series, the result of a frequency analysis in a frequency band of 0 to 20 Hz regarding a head biological signal illustrated in (b), (b) being a time-series waveform of the head biological signal, and (c) being a chart illustrating, in time series, the result of a frequency analysis in a frequency band of 0 to 10 Hz regarding the head biological signal illustrated in (b).

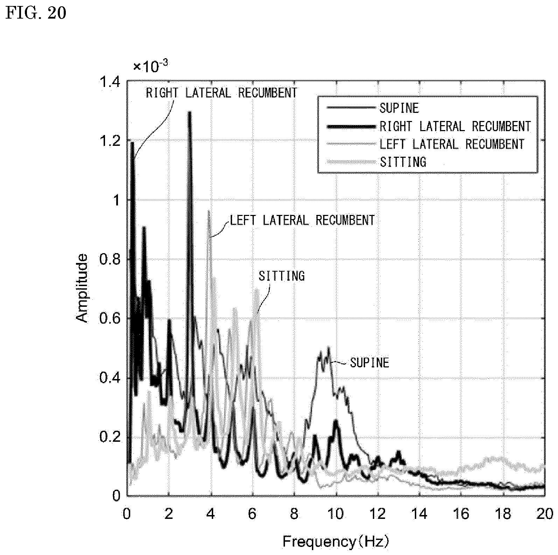

[FIG. 20] is a chart illustrating the results of frequency analyses of data in the whole measurement period of the head biological signals in the sleep experiments illustrated in FIGs. 16 to FIGs. 19 conducted while the subject is lateral recumbent, right lateral recumbent, left lateral recumbent, and sitting.

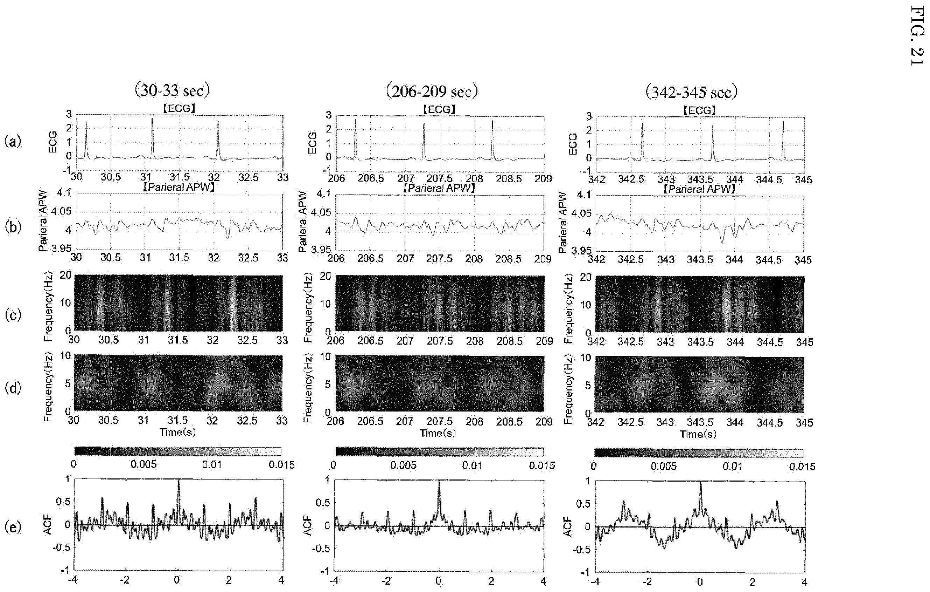

[FIGs. 21] FIGs. 21(a) to (e) are charts illustrating examples of the results of analyses by a brain activity state analysis unit using data from the sleep experiment 1 conducted while the subject is supine.

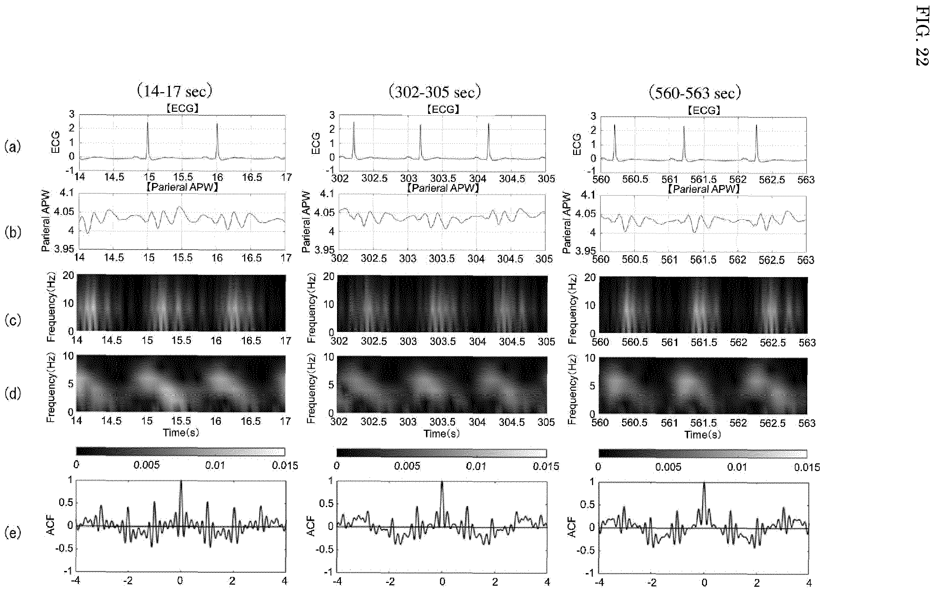

[FIGs. 22] FIGs. 22(a) to (e) are charts illustrating examples of the results of analyses by the brain activity state analysis unit using data from the sleep experiment 1 conducted while the subject is left lateral recumbent.

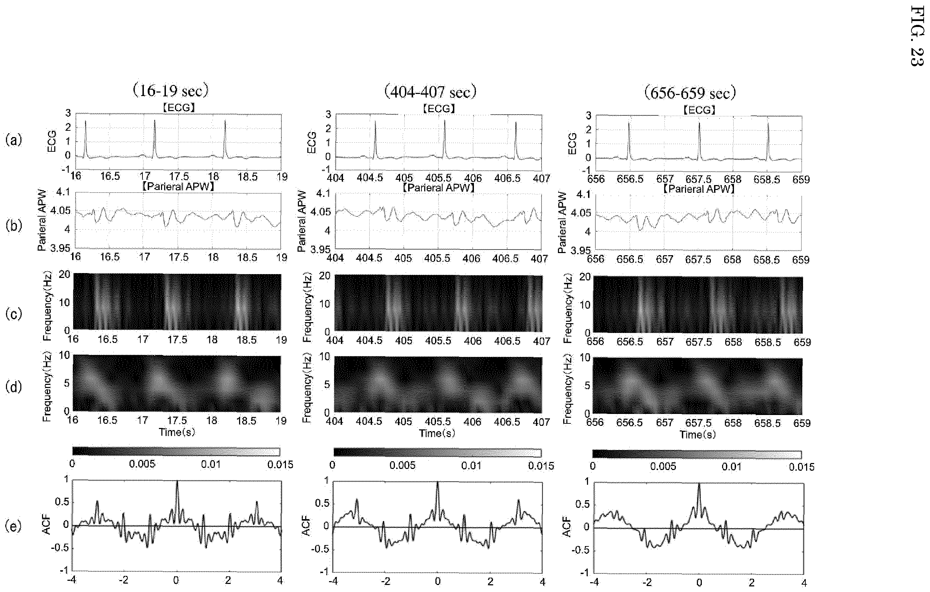

[FIGs. 23] FIGs. 23(a) to (e) are charts illustrating examples of the results of analyses by the brain activity state analysis unit using data from the sleep experiment 1 conducted while the subject is right lateral recumbent.

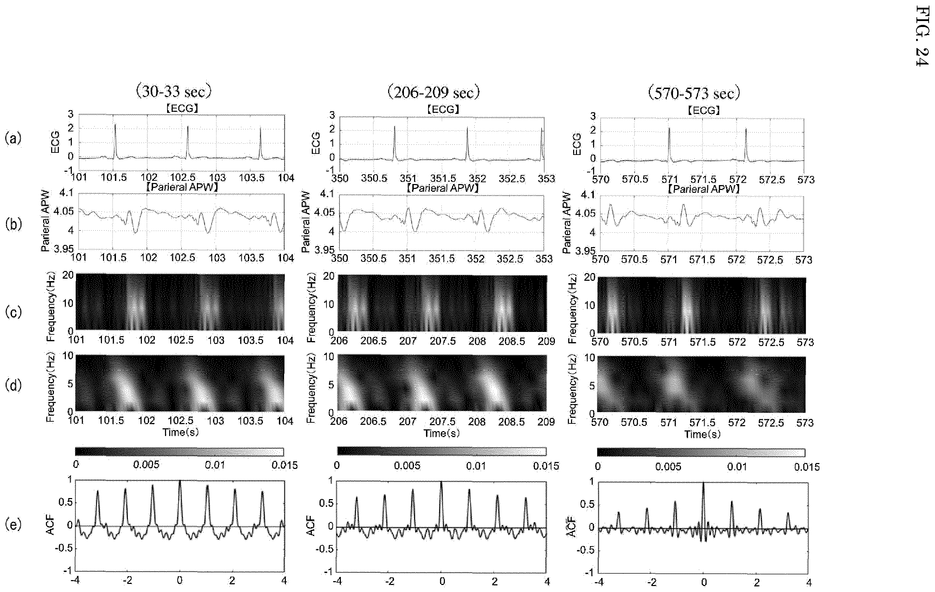

[FIGs. 24] FIGs. 24(a) to (e) are charts illustrating examples of the results of analyses by the brain activity state analysis unit using data from the sleep experiment 1 conducted while the subject is sitting.

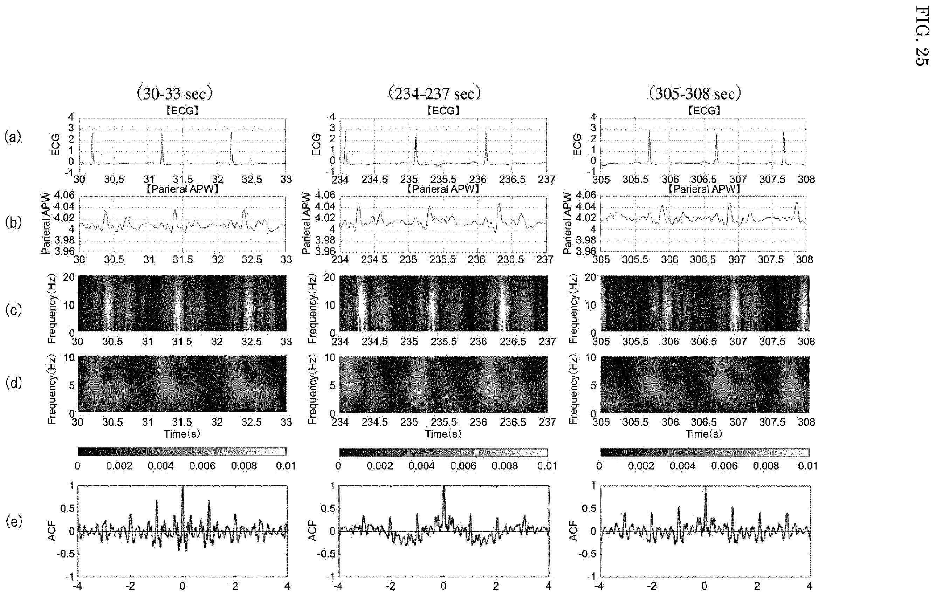

[FIGs. 25] FIGs. 25(a) to (e) are charts illustrating examples of the results of analyses by the brain activity state analysis unit using data from the sleep experiment 2 conducted while the subject is supine.

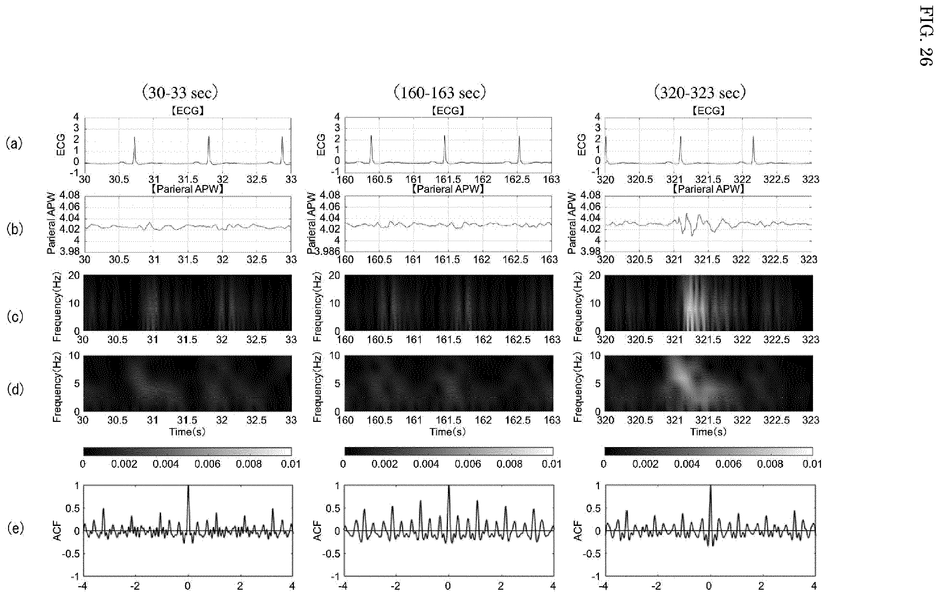

[FIGs. 26] FIGs. 26(a) to (e) are charts illustrating examples of the results of analyses by the brain activity state analysis unit using data from the sleep experiment 2 conducted while the subject is left lateral recumbent.

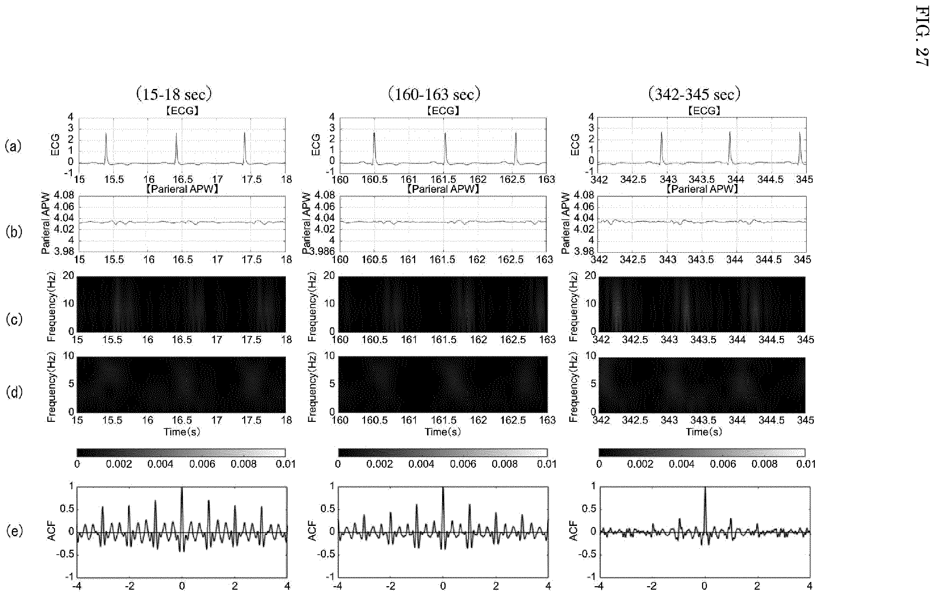

[FIGs. 27] FIGs. 27(a) to (e) are charts illustrating examples of the results of analyses by the brain activity state analysis unit using data from the sleep experiment 2 conducted while the subject is right lateral recumbent.

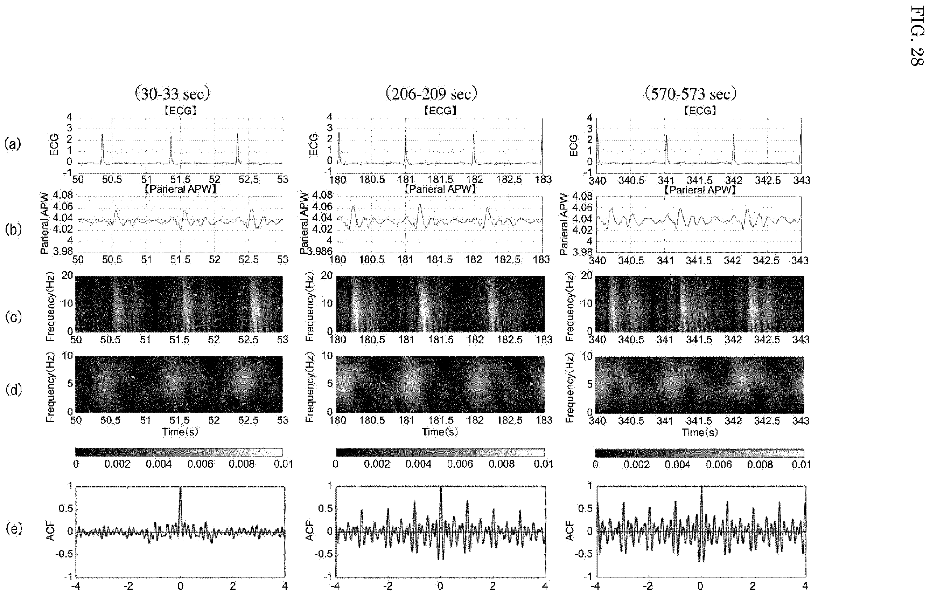

[FIGs. 28] FIGs. 28(a) to (e) are charts illustrating examples of the results of analyses by the brain activity state analysis unit using data from the sleep experiment 2 conducted while the subject is sitting.

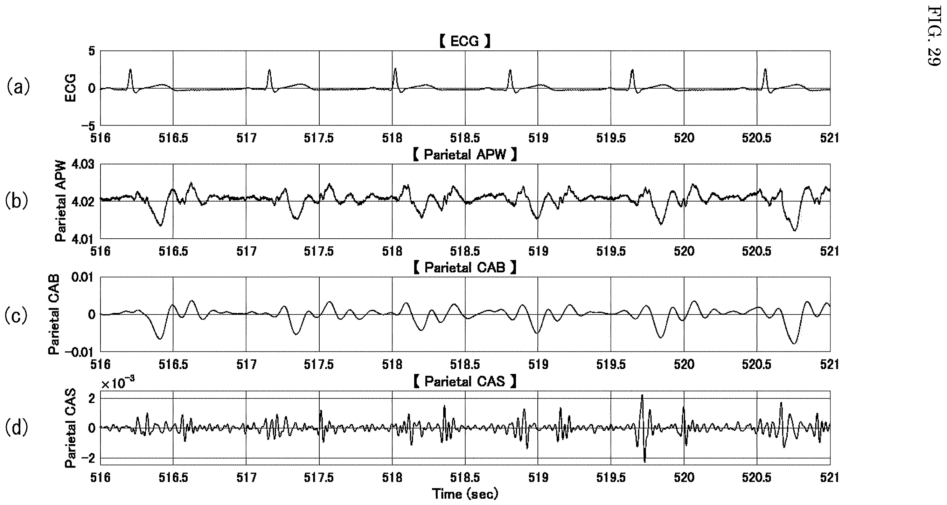

[FIGs. 29] FIGs. 29(a) to (d) are explanatory charts of an analysis method of a heartbeat-related biological signal analysis unit, (a) being a chart illustrating a waveform of an electrocardiogram, (b) being a chart illustrating a time-series waveform of a head biological signal in the same time zone as that in (a), (c) being a chart illustrating an apical beat waveform obtained by the heartbeat-related biological signal analysis unit using the head biological signal in (b), and (d) being a chart illustrating a heart sound waveform.

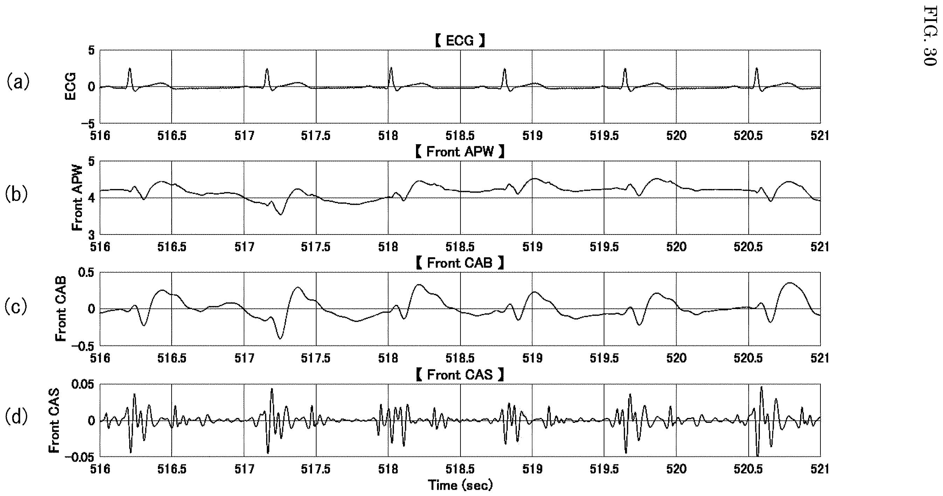

[FIGs. 30] FIGs. 30(a) to (d) are explanatory charts of an analysis method of the heartbeat-related biological signal analysis unit, (a) being a chart illustrating the waveform of the electrocardiogram, (b) being a chart illustrating a time-series waveform of a thorax biological signal in the same time zone as that in (a), (c) being a chart illustrating an apical beat waveform obtained by the heartbeat-related biological signal analysis unit using the thorax biological signal in (b), and (d) being a chart illustrating a heart sound waveform.

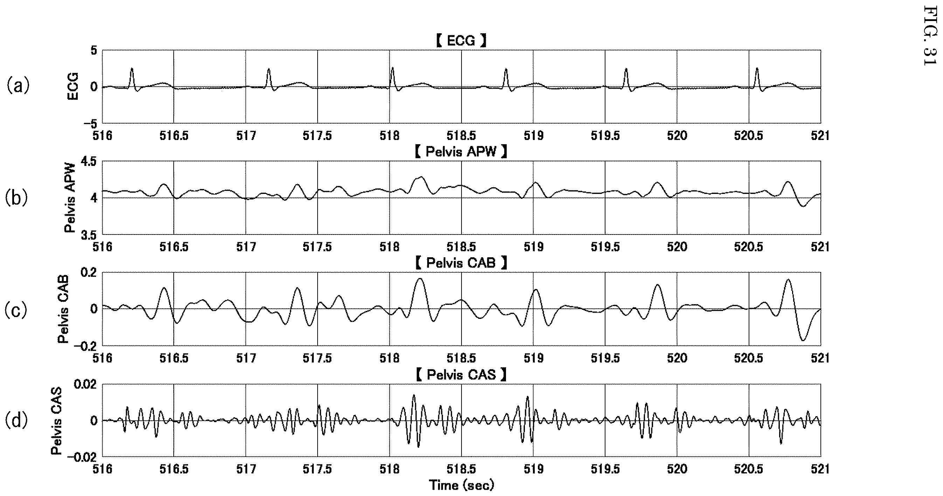

[FIGs. 31] FIGs. 31(a) to (d) are explanatory charts of an analysis method of the heartbeat-related biological signal analysis unit, (a) being a chart illustrating the waveform of the electrocardiogram, (b) being a chart illustrating a time-series waveform of a buttocks biological signal in the same time zone as that in (a), (c) being a chart illustrating an apical beat waveform obtained by the heartbeat-related biological signal analysis unit using the buttocks biological signal in (b), and (d) being a chart illustrating a heart sound waveform.

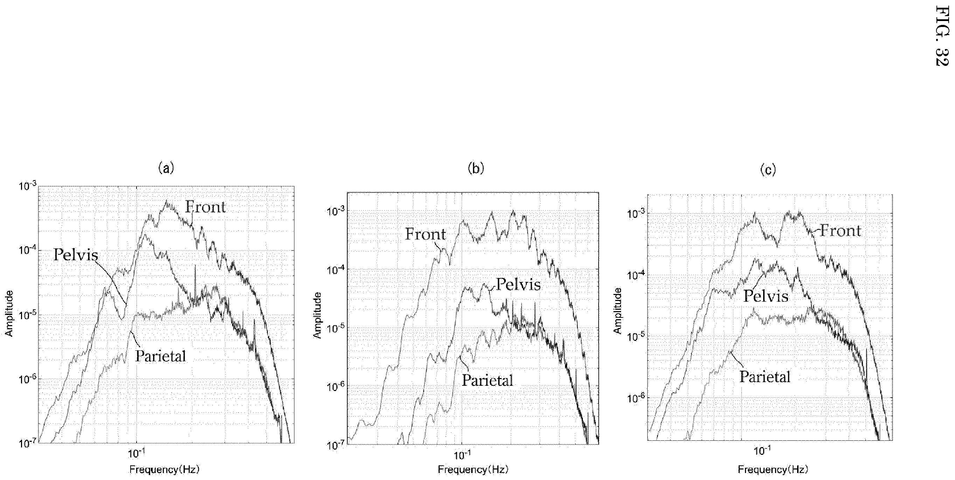

[FIGs. 32] FIGs. 32(a) to (c) are charts illustrating the frequency analysis results of the head biological signal, the thorax biological signal, and the buttocks biological signal each measured three times, from which the data are collected.

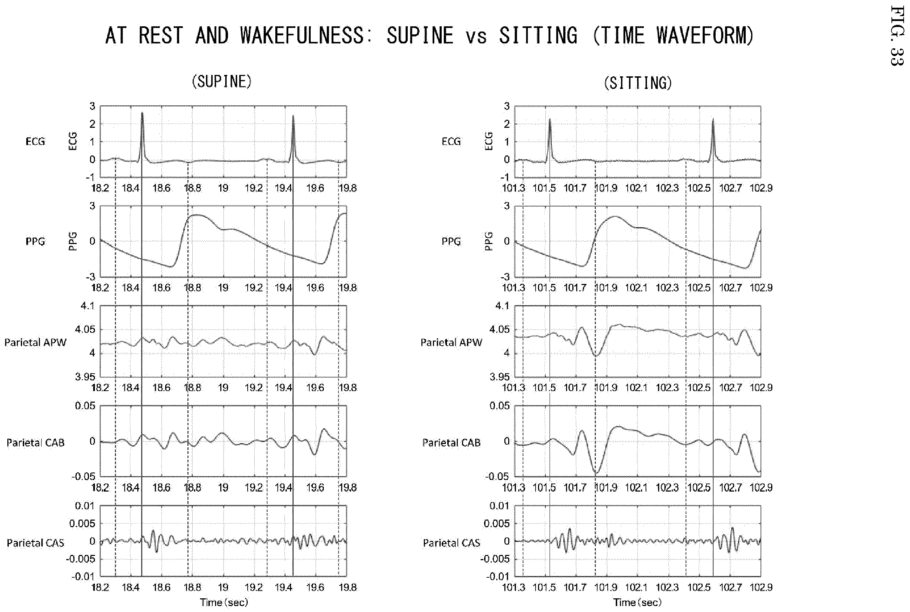

[FIG. 33] FIG. 33 is an explanatory chart of other analysis cases by the heartbeat-related biological signal analysis unit using the data obtained in the sleep experiment 2 conducted while the subject is supine and sitting and is at rest and wakefulness.

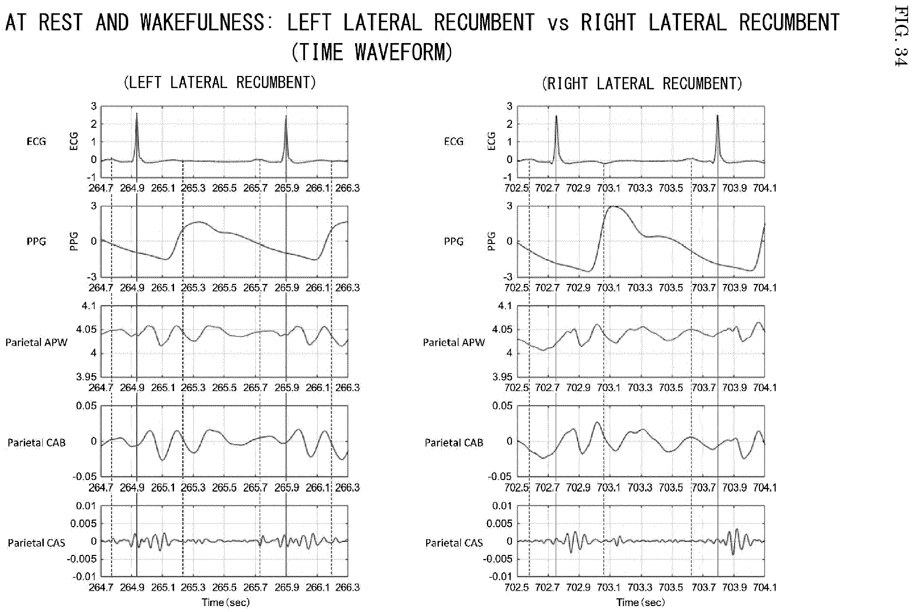

[FIG. 34] FIG. 34 is an explanatory chart of other analysis cases by the heartbeat-related biological signal analysis unit using the data obtained in the sleep experiment 2 conducted while the subject is supine and sitting and is at rest and wakefulness.

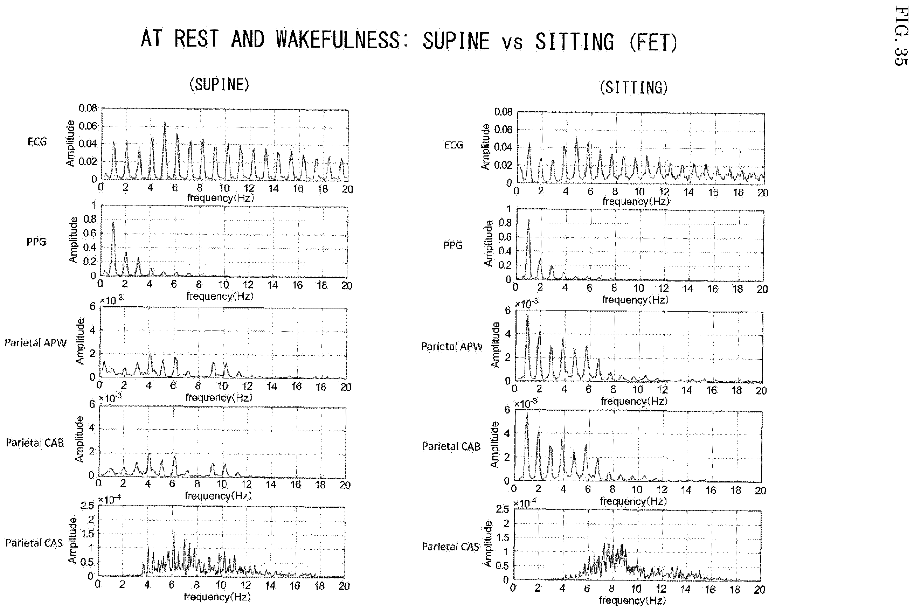

[FIG. 35] FIG. 35 is a chart illustrating the frequency analysis results of the data illustrated in FIG. 33.

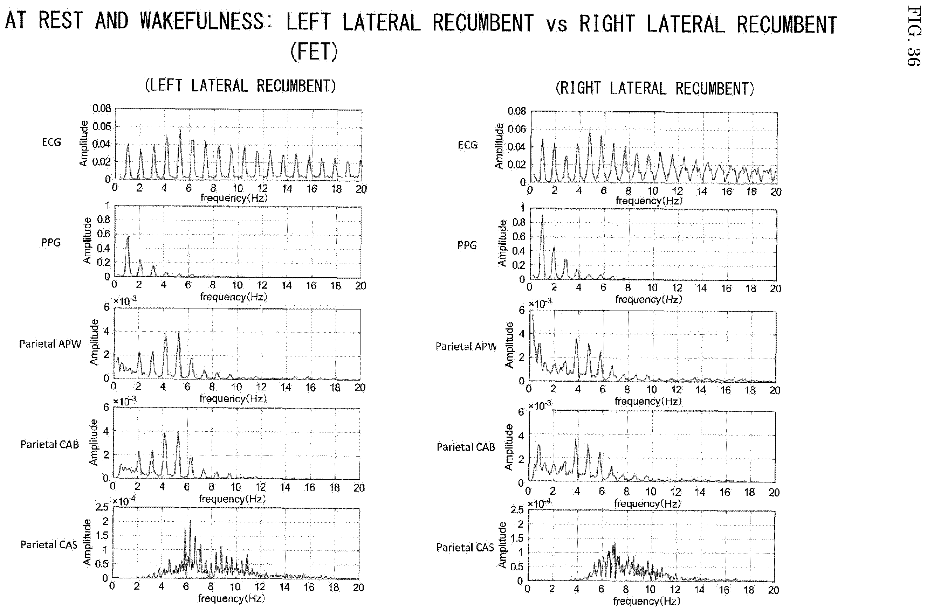

[FIG. 36] FIG. 36 is a chart illustrating the frequency analysis results of the data illustrated in FIG. 34.

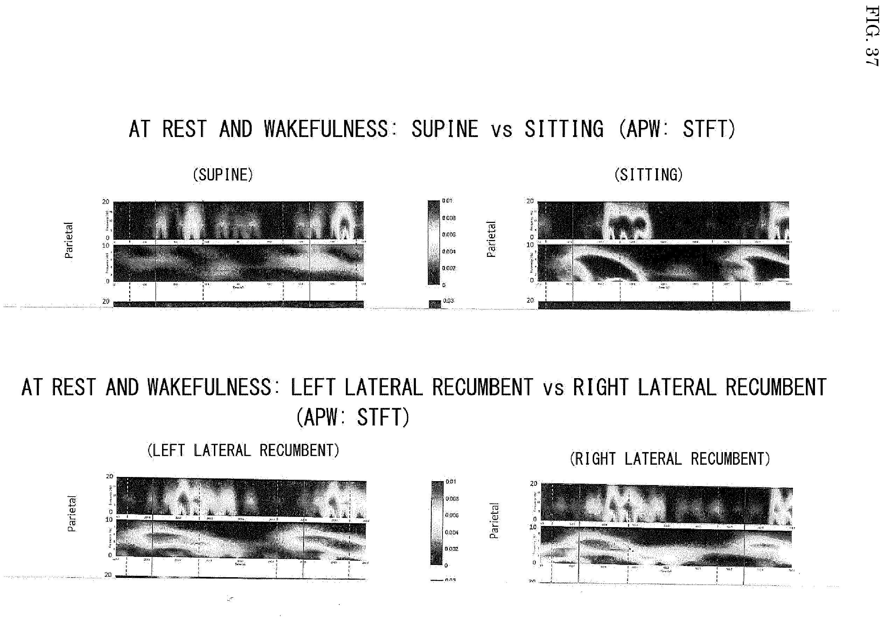

[FIG. 37] FIG. 37 illustrates STFT plots corresponding to the supine posture, the sitting posture, the left lateral recumbent posture, and the right lateral recumbent posture at rest and wakefulness, which are created using the head biological signals (Parietal APW).

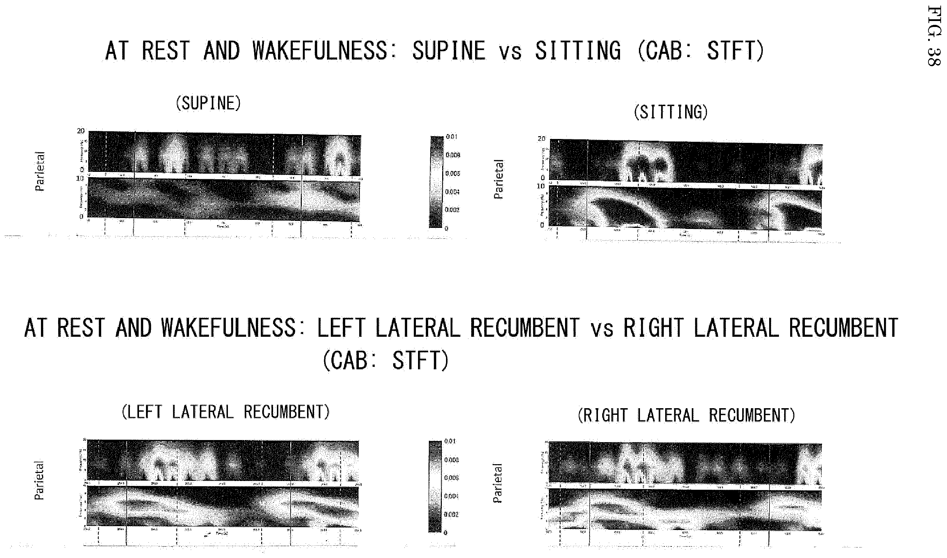

[FIG. 38] FIG. 38 illustrates STFT plots corresponding to the supine posture, the sitting posture, the left lateral recumbent posture, and the right lateral recumbent posture at rest and wakefulness, which are created using apical beat waveforms (Parietal CAB) found from the head biological signals (Parietal APW).

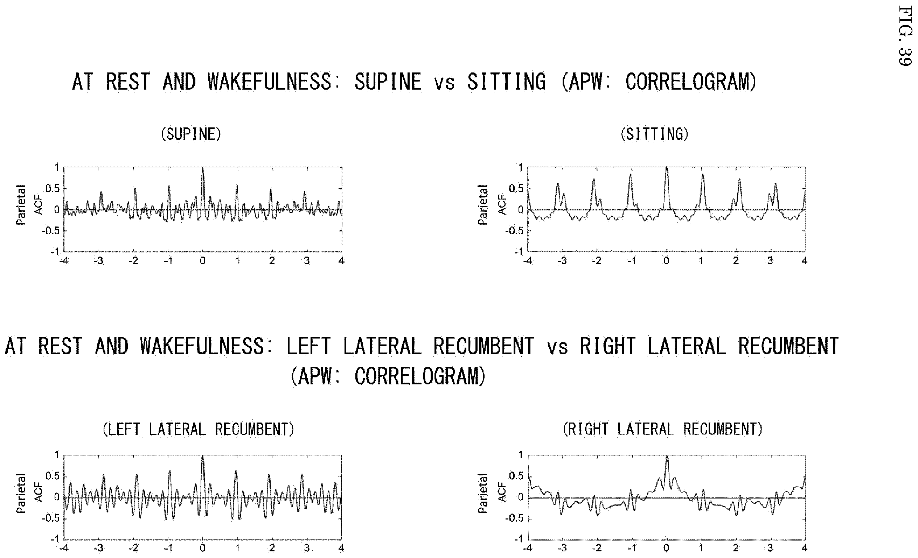

[FIG. 39] FIG. 39 illustrates correlograms corresponding to the supine posture, the sitting posture, the left lateral recumbent posture, and the right lateral recumbent posture at rest and wakefulness, which are created using the head biological signals (Parietal APW).

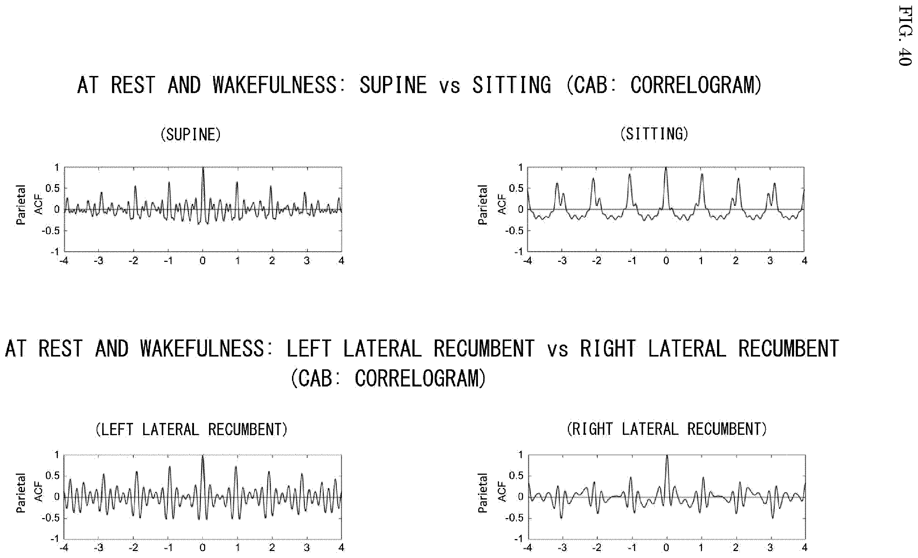

[FIG. 40] FIG. 40 illustrates correlograms corresponding to the supine posture, the sitting posture, the left lateral recumbent posture, and the right lateral recumbent posture at rest and wakefulness, which are created using the apical beat waveforms (Parietal CAB) found from the head biological signals.

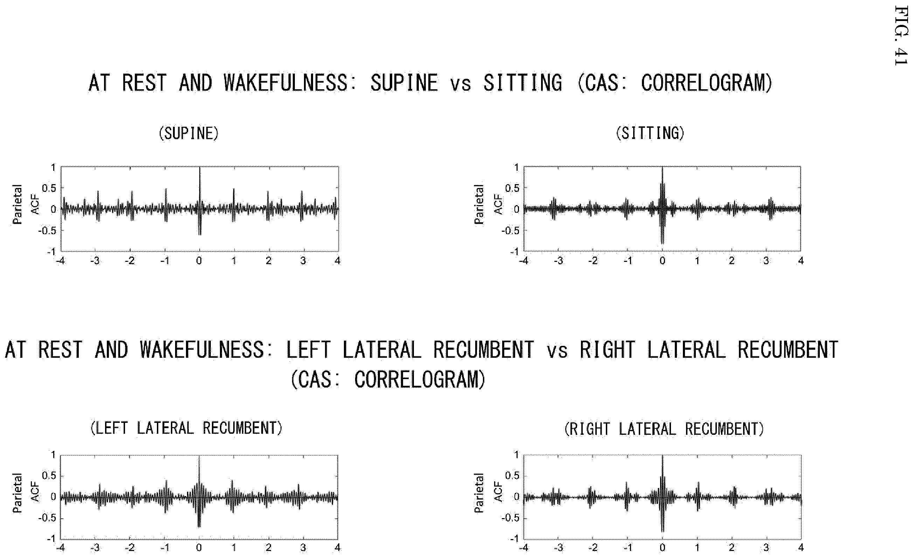

[FIG. 41] FIG. 41 illustrates correlograms corresponding to the supine posture, the sitting posture, the left lateral recumbent posture, and the right lateral recumbent posture at rest and wakefulness, which are created using heart sound waveforms (Parietal CAS) found from the head biological signals.

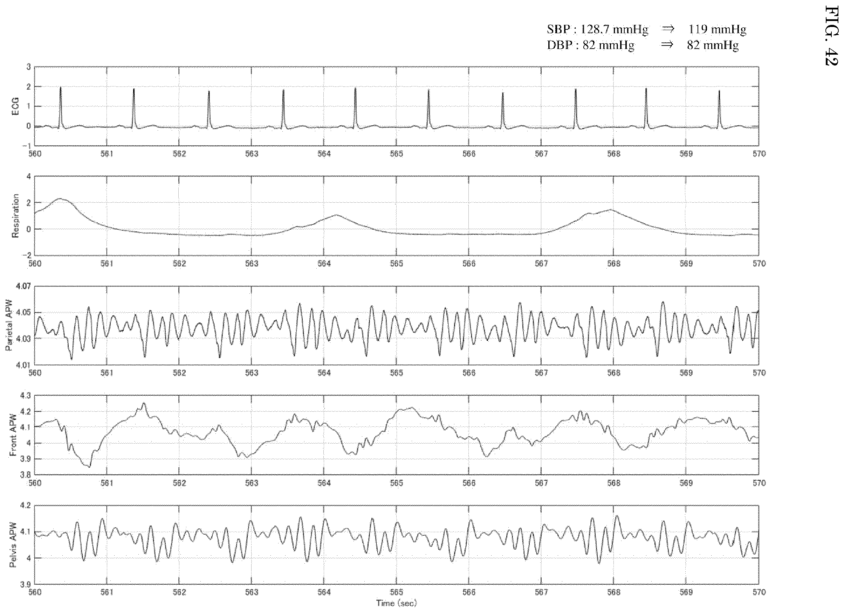

[FIG. 42] FIG. 42 is a chart illustrating part of data measured in a sleep experiment 4 while the subject is sitting and in a wake stage and illustrating time-series waveforms of an electrocardiogram, respiration, a head biological signal, a thorax biological signal, and a buttocks biological signal.

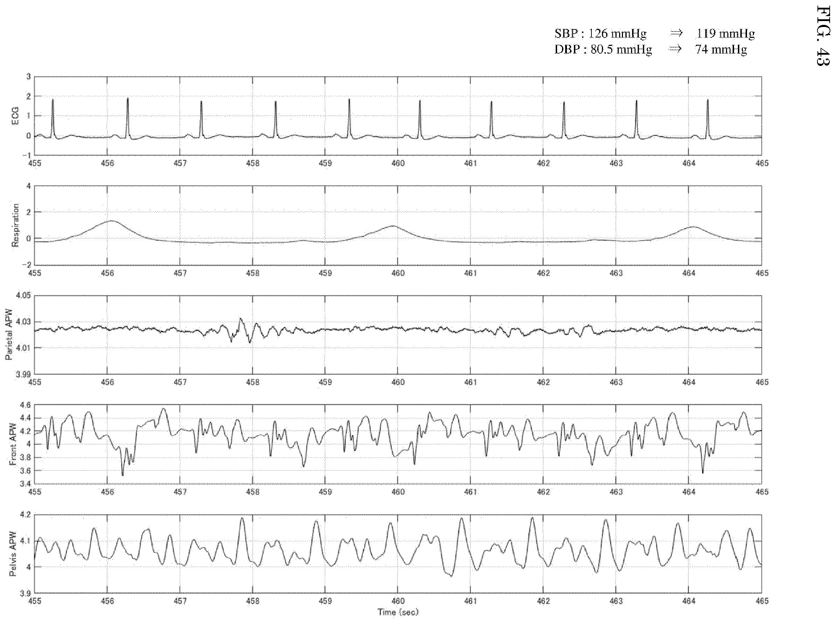

[FIG. 43] FIG. 43 is a chart illustrating part of data measured in the sleep experiment 4 while the subject is left lateral recumbent and in a first stage and illustrating time-series waveforms of an electrocardiogram, respiration, a head biological signal, a thorax biological signal, and a buttocks biological signal.

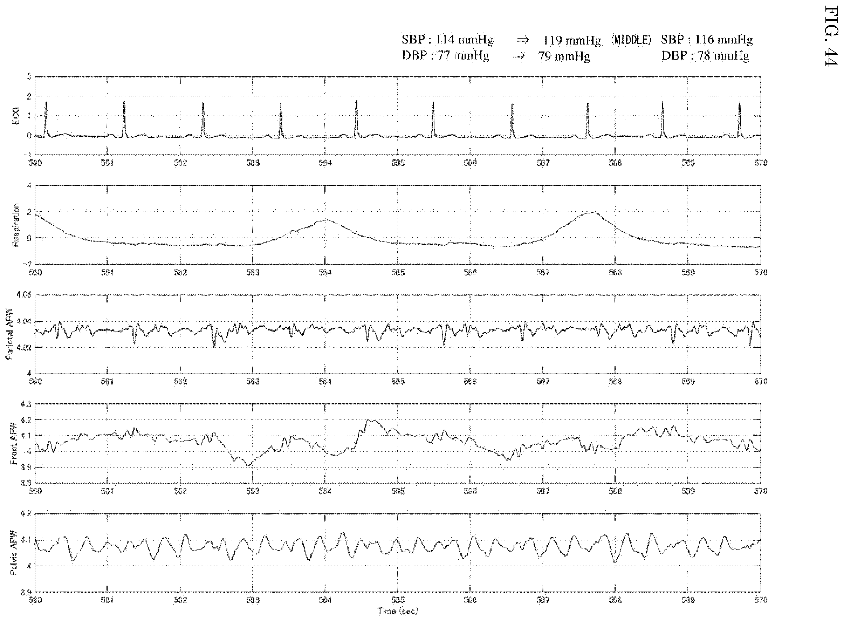

[FIG. 44] FIG. 44 is a chart illustrating part of data measured in the sleep experiment 4 on a measurement day different from that in FIG. 43 while the subject is right lateral recumbent and in the first stage and illustrating time-series waveforms of an electrocardiogram, respiration, a head biological signal, a thorax biological signal, and a buttocks biological signal.

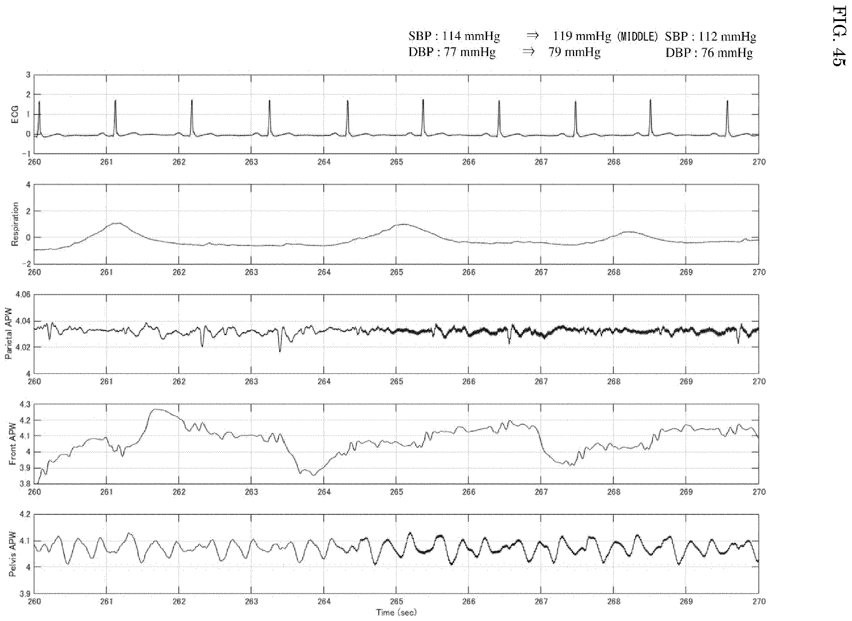

[FIG. 45] FIG. 45 is a chart illustrating part of data measured in the sleep experiment 4 on the same measurement day as that in FIG. 44 while the subject is right lateral recumbent and in a second stage and illustrating time-series waveforms of an electrocardiogram, respiration, a head biological signal, a thorax biological signal, and a buttocks biological signal.

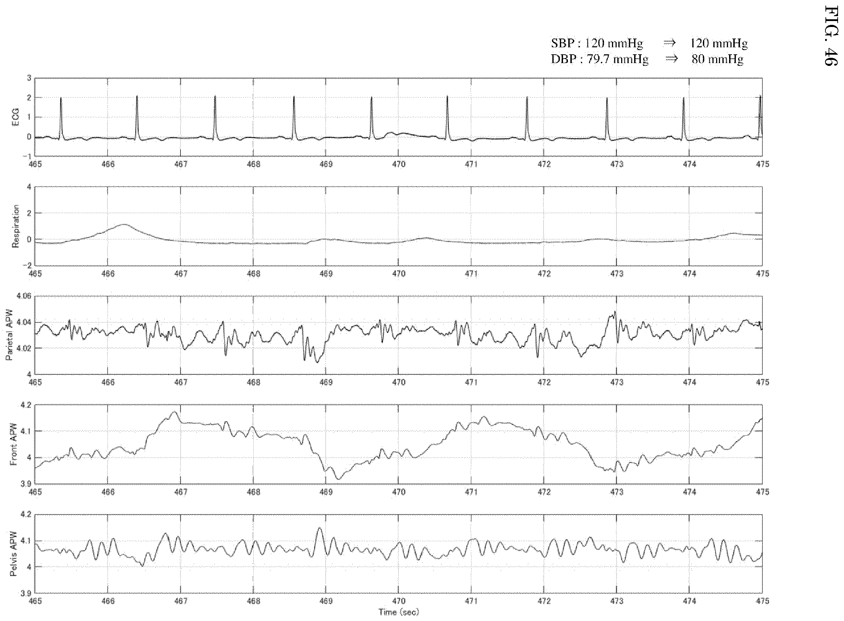

[FIG. 46] FIG. 46 is a chart illustrating part of data measured in the sleep experiment 4 on a different measurement day from that in FIG. 45 while the subject is right lateral recumbent and in the second stage and illustrating time-series waveforms of an electrocardiogram, respiration, a head biological signal, a thorax biological signal, and a buttocks biological signal.

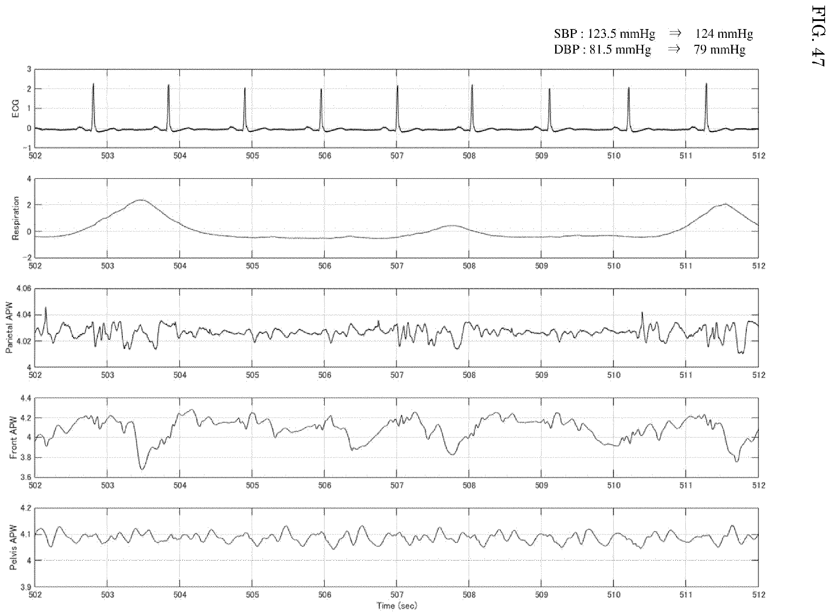

[FIG. 47] FIG. 47 is a chart illustrating part of data measured in the sleep experiment 4 on the same measurement day as that in FIG. 46 while the subject is left lateral recumbent and in a third stage and illustrating time-series waveforms of an electrocardiogram, respiration, a head biological signal, a thorax biological signal, and a buttocks biological signal.

### Modes for Carrying out the Invention

The present invention will be hereinafter described in more detail based on embodiments of the present invention illustrated in the drawings.

### (Head Biological Signal Detection Device)

First, based on FIG. 1 to FIG. 3, the configuration of a head biological signal detection device 1 used in this embodiment will be described. The head biological signal detection device 1 of this embodiment includes a head biological signal detection sensor 10 and a sensor support member 20.

The head biological signal detection sensor 10 has a three-dimensional knitted fabric 11, a housing film 12, and a microphone 13.

The three-dimensional knitted fabric 11 is formed of a pair of ground knitted fabrics 11a, 11a disposed apart from each other and a connecting yarn 11b connecting the ground knitted fabrics 11a, 11a. For example, the ground knitted fabrics 11a, 11a each can be formed to have a flat knitted fabric structure (fine meshes) continuous both in a wale direction and a course direction using yarns of twisted fibers or to have a knitted fabric structure having honeycomb (hexagonal) meshes. The connecting yarn 11b imparts certain rigidity to the three-dimensional knitted fabric 11 so that the one ground knitted fabric 11a and the other ground knitted fabric 11a are kept at a predetermined interval. Therefore, applying tension in the planar direction makes it possible to cause string vibration of the yarns of the facing ground knitted fabrics 11a, 11a forming the three-dimensional knitted fabric 11 or of the connecting yarn 11b connecting the facing ground knitted fabrics 11a, 11a. Accordingly, cardiovascular sound/vibration being a biological signal causes the string vibration and is propagated in the planar direction of the three-dimensional knitted fabric 11.

As the material of the yarns forming the ground knitted fabrics 11a, 11a or of the connecting yarn 11b of the three-dimensional knitted fabric 11, various materials are usable, and examples thereof include synthetic fibers and regenerated fibers such as polypropylene, polyester, polyamide, polyacrylonitrile, and rayon, and natural fibers such as wool, silk, and cotton. These materials each may be used alone or any combination of these may be used. Preferably, the material is a polyester-based fiber represented by polyethylene terephthalate (PET), polybutylene terephthalate (PBT), and the like, a polyamide-based fiber represented by nylon 6, nylon 66, and the like, a polyolefin-based fiber represented by polyethylene, polypropylene, and the like, or a combination of two kinds or more of these fibers. Further, the shapes of the yarns forming the ground knitted fabrics 11a, 11a and of the connecting yarn 11b are not limited either, and any of round cross-section yarns, modified cross-section yarns, hollow yarns, or the like may be used. Carbon yarns, metallic yarns, and so on are also usable.

The following products are usable as the three-dimensional knitted fabric 11, for instance.
(a) product number: 49013D (manufactured by Sumie Textile Co., Ltd.), 10 mm thickness
   material:
   front-side ground knitted fabric ... twisted yarn of two polyethylene terephthalate fiber false twisted yarns with 450 decitexes/108 f
   rear-side ground knitted fabric ... twisted yarn of two polyethylene terephthalate fiber false twisted yarns with 450 decitexes/108 f
   connecting yarn ................. polytrimethylene terephthalate monofilament with 350 decitexes/1 f
(b) product number: AKE70042 (manufactured by Asahi Kasei Corporation), 7 mm thickness
(c) product number: T28019C8G (manufactured by Asahi Kasei Corporation), 7 mm thickness

The three-dimensional knitted fabric 11 is covered with the housing film 12. In this embodiment, the housing film 12 is composed of two films made of a synthetic resin, they are arranged to cover the front surface and the rear surface of the three-dimensional knitted fabric 11, and peripheral edge portions of the films are bonded by welding. Consequently, the three-dimensional knitted fabric 11 is sealingly housed in the housing film 12.

A case 14 is attached to an outer side of the housing film 12, and the microphone 13 is disposed in the case 14. A gel 15 as an external disturbance mixture preventing member is filled in the case 14 to surround the microphone 13. The case 14 is made of a synthetic resin and has the function of preventing acoustic vibration propagated to the microphone 13 from spreading out, and the gel 15 inhibits external vibration from being captured by the microphone 13. A cord 13a that carries detected acoustic vibration data is connected to the microphone 13. Further, from a viewpoint of noise countermeasure, the aforesaid case 14 and gel 15 are preferably provided, but in consideration of the arrangement on the head, a configuration giving a higher priority to even a slight weight reduction is also adoptable, that is, the microphone 13 may be loaded in the housing film 12, without the case 14 and the gel 15 being provided.

The sensor support member 20 is a member with which the head biological signal detection sensor 10 is supported at a predetermined position of the head. In the head biological signal detection sensor 10, a surface, of the housing film 12 housing the three-dimensional knitted fabric 11, opposite to the surface on which the case 14 loaded with the microphone 13 is stacked is an abutting surface 10a that abuts on the head. Therefore, the sensor support member 20 supports the head biological signal detection sensor 10 such that the head biological signal detection sensor 10 is located at the predetermined position, with the abutting surface 10a abutting on the head.

In this embodiment, as the sensor support member 20, a cap type is used. Specifically, it has a crown 21 and a brim portion 22. In this embodiment, it is of a cap type having the brim portion 22 only along a front edge side of the crown 21, but the type of the cap is not limited as long as it is one in whose crown 21 the head biological signal detection sensor 10 can be disposed, such as a hat type having the brim portion 22 formed all around the peripheral edge portion of the crown 21.

In the crown 21 of the cap-type sensor support member 20 of this embodiment (hereinafter, "cap-type sensor support member"), the head biological signal detection sensor 10 is disposed at such a position that part of the abutting surface 10a, preferably, the vicinity of its center, abuts on the parietal region. To detect a head biological signal while a subject wears the cap-type sensor support member 20, the connecting yarn 11b is caused to sag so that the interval between the pair of facing ground knitted fabrics 11a, 11a in the three-dimensional knitted fabric 11 housed in the housing film 12 forming the head biological signal detection sensor 10 becomes narrower than that under no load. The interval between the pair facing ground knitted fabrics 11a, 11a after the sagging is preferably a 40 to 95% interval, and more preferably a 70 to 90% interval of a reference which is an interval between the pair of facing ground knitted fabrics 11a, 11a when no load is applied to the three-dimensional knitted fabric 11 housed in the housing film 12.

To cause such sagging of the three-dimensional knitted fabric 11 and keep this state during the measurement of the head biological signal, a worn state maintaining structure capable of maintaining the sagging state is provided in the cap-type sensor support member 20. In this embodiment, as the worn state maintaining structure, a fastening structure 23 is provided at a position that is on the lower edge 21a of the crown 21 and where a hatband (sweatband) is provided, to keep the position by being in close contact with the periphery of the head.

The fastening structure 23 may be an elastic member such as rubber in a belt shape or strap shape disposed along the lower edge 21a of the crown 21, a structure similarly disposed along the lower edge 21a and composed of, for example, two belt-shaped members, with the total length of the two belt-shaped members being adjustable by changing the engagement position of the two belt-shaped members (see FIG. 2(c)), or the like. It may be a combination of the elastic member and the belt-shaped members.

The crown 21 forming the cap-type sensor support member 20 can be formed of a cloth material typically used for forming caps, but is preferably formed of the same material as the aforesaid three-dimensional knitted fabric 11 forming the head biological signal detection sensor 10. Forming the crown 21 of the three-dimensional knitted fabric can reduce the input of unnecessary external sound/vibration to the head biological signal detection sensor 10.

A head biological signal is sound/vibration generated by cerebral blood flow, but since cerebral blood flow is transmitted through blood vessels in the skull, its sound/vibration is weak. Therefore, it is difficult to detect this sound/vibration from the surface of the head, but in this embodiment, the three-dimensional knitted fabric 11 forming the head biological signal detection sensor 10 is caused to sag and the fastening structure 23 maintains the sagging during the measurement as described above. Consequently, as the head moves due to body motion or the like, microvibration easily occurs in the yarns and the fibers forming the three-dimensional knitted fabric 11 and in the housing film 12, and the microvibration works as noise. As a result, stochastic resonance is generated between the noise and the head biological signal which is sound/vibration generated by the cerebral blood flow, making it possible to detect the head biological signal.

Here, FIGs. 5(a) to (h) illustrate time-series waveforms of an electrocardiogram, a finger plethysmogram, respiration, a head biological signal, a thorax biological signal, a dorsal biological signal, a lumbar biological signal, and a buttocks biological signal in order, and the head biological signal detection device 1 of this embodiment is capable of efficiently using the stochastic resonance phenomenon by having the cap-type sensor support member 20 as described above, and even though it is attached to the outer side of the skull, the head biological signal can be captured with high sensitivity similarly to biological signals of the other regions as illustrated in FIG. 5(d). Note that reference sign 1000 in FIG. 1 denotes a biological signal detection sensor having the same configuration as that of the head biological signal detection sensor 10, and a signal obtained when this biological signal detection sensor 1000 is attached to the thorax is the thorax biological signal, a signal obtained when it is attached to the back is the dorsal biological signal, a signal obtained when it is attached to the lumbar is the lumbar biological signal, and a signal obtained when it is placed under the buttocks is the buttocks biological signal.

### (Biological State Estimation Device)

Next, a biological state estimation device 100 will be described. As illustrated in FIG. 4, the biological state estimation device 100 of this embodiment has a head biological signal analysis unit 110. The head biological signal analysis unit 110 receives and analyzes a head biological signal from the head biological signal detection sensor 10, estimates a predetermined state of a person together with an occurrence instant of this state, and outputs the estimation result.

The head biological signal analysis unit 110 has a brain activity state analysis unit 120 and a heartbeat-related biological signal analysis unit 130.

The brain activity state analysis unit 120 has a sleep stage identifying unit 121 and a wakefulness degree change index estimation unit 122. The sleep stage identifying unit 121 frequency-analyzes the head biological signal obtained from the head biological signal detection sensor 10 to find, in time series, the main frequency band of the head biological signal and based on the main frequency band, identifies a sleep stage out of sleep stages including a wake stage, a drowsiness stage to a deep sleep stage, and a REM sleep stage. For example, FIG. 16(b) illustrates an example of a time-series waveform of the head biological signal obtained from the head biological signal detection sensor 10, and the results of the frequency analysis of this are the graphs illustrated in FIG. 16(a) and FIG. 16(c). FIG. 16(a) illustrates, in time series, the results of frequency analyses in analysis sections regarding the time-series waveform of the head biological signal illustrated in FIG. 16(b), with each analysis section being set to 128 points (0.128 seconds) and with the starting point of the analysis section being slid by 12 points (0.012 seconds) each time. FIG. 16(c) illustrates, in time series, the results of frequency analyses in analysis sections regarding the time-series waveform of the head biological signal illustrated in FIG. 16(b), with each analysis section being set to 512 points (0.512 seconds) and with the starting point of the analysis section being slid by 51 points (0.051 seconds) each time. In both of these, the X-axis represents time, the Y-axis represents frequency, and the Z-axis represents amplitude (gain). Based on a temporal change of the main frequency band in the frequency analysis results, a sleep stage is identified. The main frequency band refers to a frequency band having a relatively high gain, and for example, near 80 to 140 seconds in FIG 16(a), 3 to 20 Hz is the main frequency band.

Sleep stages are classified into a wake stage (Stage W), a drowsiness stage + a very light sleep stage = Stage 1, a light sleep stage (Stage 2), a moderately deep sleep stage (Stage 3), a deep sleep stage (Stage 4), and a REM sleep stage (Stage REM) according to the international criteria that use brain waves.

Here, brain waves are classified into an α wave: 8 to 13 Hz, a β wave: 14 to 30 Hz, a θ wave: 4 to 7 Hz, and a δ wave: 0.5 to 3 Hz. How these brain waves appear, which differs depending on the depth of sleep, can indicate the depth of sleep, but in this embodiment, the sleep stage is estimated using the head biological signal obtained from the head biological signal detection sensor 10 instead of using a brain wave measured with an electroencephalograph. The head biological signal detection sensor 10 of this embodiment captures information on intracranial cerebral blood flow as will be described later. The state of the intracranial cerebral blood flow is the state of blood that has come from the heart through the internal carotid arteries and the external carotid arteries to flow in blood vessels in the brain, reflects the supply speed of oxygen serving as energy for brain activities, and is associated with the wakefulness degree and sleepiness. Therefore, from information in its sound/vibration, it is thought that the depth of sleep can be found.

Therefore, when a variation in the main frequency band of the time-series waveform of the head biological signal obtained from the head biological signal detection sensor 10 was found using the sleep stage identifying unit 121, the present inventor found that a subject's state change estimated from a change of a frequency band substantially the same as each of the frequency bands of the brain waves is substantially the same as a sleep stage determined using the brain wave as described above.

### (Sleep Experiments)

Sleep experiments 1 to 3 were conducted on the same subject on different experiment days.

In the sleep experiment 1, the subject drank alcohol (Japanese sake 100 cc) before sleeping on the preceding day of the experiment and was subjected to the experiment while having no subjective fatigue feeling at the start of the experiment next morning. In the sleep experiments 2, 3, the subject did not drink alcohol before sleeping on the preceding days of the experiments and was subjected to the experiments while having a subjective fatigue feeling at the start of the experiments next morning. Further, all of the sleep experiments 1 to 3 were conducted while the subject took different supine, left lateral recumbent, right lateral recumbent, and sitting postures. In the experiments, head biological signals were obtained from the head biological signal detection device 1 while the subject took these postures, and were frequency-analyzed by the sleep stage identifying unit 121 in the above-described manner, and the results were shown in time series. Note that, in each of FIGs. 6 to FIGs. 13 which are the results of the sleep experiments 1, 2, (a) illustrates a time-series change in a frequency band of 0 to 20 Hz, and (b) illustrates a time-series change in a frequency of 0 to 10 Hz.

### (Sleep Experiment 1)

FIGs. 6 illustrate data from the sleep experiment conducted while the subject is supine, which indicate a resting state (state without any fatigue feeling) to a state with a low wakefulness degree (low wakefulness state). In the time-series waveforms of the frequency analysis results of the head biological signal, α waves (resting), intermittent α waves (relaxation), and low-amplitude fast waves are seen, and various waves with faster frequencies than those in the other sleep stages appear in mixture, indicating a wake stage (Stage W).

FIGs. 7 illustrate data from the sleep experiment conducted while the subject is right lateral recumbent. They indicate a transition from the stage with a low wakefulness degree to the first stage (Stage 1).

The brain wave feature at the transition from the wake stage to the first stage is that the α waves in the wake stage decrease in amplitude, their continuity gradually worsens, they only appear with breaks and finally completely disappear, and then a low-amplitude pattern unique to the first stage appears. At the same time, a single low-amplitude θ wave starts appearing or several low-amplitude θ waves continuously start appearing, at the center region, the frontal region, the parietal region (temporal region), and so on, and a low-amplitude fast wave of around 20 Hz also appears, so that the whole waveform of the brain wave looks as if rippling. In the first stage, the α wave disappears, and a brain pattern appears where waves with relatively low amplitudes and various frequencies are mixed due to the presence of a flat waveform, a low-amplitude θ wave, a fast wave, and a vertex sharp wave, and in particular, waves in a 2 to 7 Hz (6 to 7 Hz is the θ wave) range are dominant. This corresponds to a stage where a person feels sleepy and dozes off.

The first stage lasts for a relatively short time of one to seven minutes, and in the latter half of the first stage, the vertex sharp wave sometimes appears but it appears in complex with a high-amplitude 2 to 7 Hz wave.

At the time of the transition from the wake stage to the first stage, an instant when the α wave reduces to 50% or less is determined as the first stage.

The time-series waveforms of the frequency analysis results of the head biological signal in FIGs. 7 present substantially the same features as the aforesaid brain wave features at the transition from the wake stage to the first stage and that in the first stage.

Next, at the time of the transition of sleep from the first stage to the second stage (Stage 2), in the case of the brain wave, a rippling waveform appears at the time of the transition from the first stage to the second stage, and thereafter, at the vertex region, a high-amplitude vertex sharp wave like a lump appears. The vertex sharp wave often appears singly but two to three sometimes continuously appear. The criterion for the second stage is the appearance of a sleep spindle wave and a K-complex wave resembling the vertex sharp wave and the absence of a high-amplitude slow wave. The K-complex wave lasts at the vertex region for 0.5 seconds or more. Then, after the around 14-Hz lump/spindle mixture stage, the vertex sharp wave disappears, and only the 14-Hz spindle wave stably appears. As the sleep deepens, a spindle wave of 10 to 12 Hz also appears. A spindle wave of the aged has a smaller amplitude than that of a young person. In addition to the spindle wave and the K complex wave, low-amplitude θ wave, δ wave, and so on are included as background brain waves.

The time-series waveforms of the frequency analysis results of the head biological signal in FIGs. 8(a), (b) are data from the sleep experiment conducted while the subject is left lateral recumbent, which present substantially the same features as the aforesaid brain wave features presented when the second stage is reached after the transition from the first stage to the second stage, and it is seen that, from FIGs. 8(a), (b), a sleep stage can be estimated.

FIGs. 9 illustrate data from the sleep experiment conducted while the subject is sitting, where the REM sleep stage is mainly present. The brain wave feature in the REM sleep stage is that relatively low-amplitude brain waves with mixed frequencies and a sawtooth wave in the θ wave band appear, and the appearance of an α wave also increases. However, this α wave is lower in frequency than the α wave in the wake stage by 1 to 2 Hz. The time-series waveforms of the frequency analysis results of the head biological signal in FIGs. 9 present a tendency similar to the frequency change of the brain wave in the REM sleep, and thus a sleep stage can be estimated as the REM sleep stage.

The brain wave feature in the third stage (Stage 3) is that a 2-Hz or lower slow wave whose amplitude is increased to about 1.5 times occupies 20 to 50%. This is distinguished from the autonomous K-complex wave in the second stage. The frequency of the spindle wave decreases to around 10 Hz.

The brain wave feature in the fourth stage (Stage 4) is that higher-amplitude slow waves of 2 Hz or lower begin to appear to occupy more than 50%. These high-amplitude slow waves are considered to represent functional depression of the cerebrum.

Note that, in the sleep experiments 1 to 3, the state of the subject did not shift to the third stage or the fourth stage, but it is of course possible to determine the third stage and the fourth stage by applying the brain wave features in the third stage and the fourth stage to the time-series waveforms of the frequency analysis results of the head biological signal.

### (Sleep Experiment 2)

FIGs. 10 to FIGs. 13 illustrate time-series waveforms of the frequency analysis results of head biological signals in the sleep experiment 2.

FIGs. 10 illustrate data from the sleep experiment conducted while the subject is supine, which present a phenomenon of the resting state (state with a fatigue feeling) and present the feature in the wake stage (Stage W).

FIGs. 11 illustrate data from the sleep experiment conducted while the subject is left lateral recumbent, which present the aforesaid feature in the first stage (Stage 1) which is the stage in which a person feels sleepy and dozes off. However, as compared with the data in FIGs. 7 of the sleep experiment 1 presenting the feature in the same first stage, a gain (amplitude) is small. It can be inferred that this is because sleep is relatively deep even in the first stage since the subject had a fatigue feeling.

FIGs. 12 illustrate data from the sleep experiment conducted while the subject is right lateral recumbent, which present the aforesaid features in the first stage to the second stage (Stage 2), but a gain (amplitude) is smaller than those of the data in FIGs. 8 in the sleep experiment 1 similarly presenting the features in the first stage to the second stage. It can be inferred that this is also because the sleep is deeper even though the sleep is in the same second stage.

FIGs. 13 illustrate data from the sleep experiment conducted while the subject is sitting, which present the feature in the wake stage (Stage W). It can be said that the subject has recovered from the fatigue and is at rest because his/her sleep is relatively deep even in the first stage and the second stage in FIGs. 11 and FIGs. 12.

FIGs. 14(a) to (d) are charts illustrating the frequency analysis results of all the time-series waveforms of the head biological signals in the sleep experiment 1 conducted while the subject is supine, left lateral recumbent, right lateral recumbent, and sitting. In SUPINE in FIG. 14(a), gains of a δ wave and a θ wave of 0.5 to 7 Hz are relatively large, while gains of an α wave of 8 to 12 Hz are also relatively large, which presents the feature in the wake stage at rest. In LEFT LATERAL RECUMBENT in FIG. 14(b), a θ wave of 4 to 7 Hz mainly occupies, which presents the feature in the first stage. In RIGHT LATERAL RECUMBENT in FIG. 14(c), a δ wave of 0.5 to 3 Hz mainly occupies, which presents the feature in the second stage. In SITTING in FIG. 14(d), a gain of a θ wave is large and an α wave of around 8 Hz also appears, which presents the feature in the REM sleep stage.

FIGs. 15(a) to (d) are charts illustrating the frequency analysis results of all the time-series waveforms of the head biological signals in the sleep experiment 2 conducted while the subject is supine, left lateral recumbent, right lateral recumbent, and sitting. SUPINE, LEFT LATERAL RECUMBENT, and RIGHT LATERAL RECUMBENT illustrated in FIGs. 15(a) to (c) are the frequency analysis results, which present the features in the wake stage at rest, the first stage, and the second stage respectively. In the frequency analysis result in SITTING in FIG. 15(d), gains of a δ wave and a θ wave of 0.5 to 7 Hz are relatively large, and a gain of an α wave of around 10 Hz is also relatively large, which presents the feature in the wake stage.

### (Sleep Experiment 3)

FIGs. 16 to FIGs. 19 illustrate the results of the sleep experiment 3. FIGs. 16 are data from the sleep experiment conducted while the subject is supine, and the subject begins to doze off at 300 seconds but is awake before this. However, as is seen from "HIGH" and "LOW" written in the upper column of FIG. 16(b), states with high wakefulness degrees and states with low wakefulness degrees appear. Near 50 seconds, near 80 to 140 seconds, and near 160 to 260 seconds from the start of the measurement, it is seen from FIG. 16(a) that the main frequency bands are an α wave and a β wave, and from the time-series waveforms in FIGs. 16(a), (c), a state with a high wakefulness degree is seen. Near 60 to 80 seconds, near 90 to 110 seconds, and near 260 to 280 seconds, an α wave of around 10 Hz mainly occupies, from which it is seen that the wakefulness degree is low. In the "DROWSY" state at and after 300 seconds, a θ wave and a δ wave of 2 to 7 Hz are mixed as illustrated in FIG. 16(c), from which it is seen that the subject feels sleepy.

FIGs. 17 are data from the sleep experiment conducted while the subject is left lateral recumbent. In FIGs. 17(a), (c), an α wave mainly occupies until the vicinity of 80 seconds after the start of the experiment, but thereafter, the α wave reduces. Near 150 seconds, a β wave appears, from which it can be read that the subject temporarily becomes awake. At and after around 150 seconds, a δ wave and a θ wave mainly occupy, from which it can be inferred that the second stage is entered.

FIGs. 18 are data from the sleep experiment conducted while the subject is right lateral recumbent, where, near 100 seconds, near 240 seconds, near 270 seconds, and near 350 seconds, spindle waves of 12 to 14 Hz appear, and at substantially the same instants, θ waves and δ waves of 2 to 7 Hz also appear, from which it can be inferred that a period from 100 seconds on corresponds to the second stage.

FIGs. 19 are data from the sleep experiment conducted while the subject is sitting. From 20 seconds on after the start of the measurement, the resting state where an α wave mainly occupies continues, and near 170 seconds, near 200 seconds, and near 210 seconds, θ waves and δ waves of 2 to 7 Hz are mixed as illustrated in FIG. 19(c), which indicates that the first stage is reached. Near 260 seconds, a spindle wave appears, and it can be inferred that the stage shifts to the second stage thereafter.

FIG. 20 is a chart illustrating the frequency analysis results of all the time-series waveforms of the head biological signals obtained while the subject is supine, right lateral recumbent, left lateral recumbent, and sitting, which are illustrated in (b) of FIGs. 16 to FIGs. 19. As illustrated in this drawing, in SUPINE, gains of a δ wave and a θ wave of 0.5 to 7 Hz are relatively large, while a gain of an α wave of 8 to 12 Hz is also large. From this, it is seen that the wakeful time is long, while a certain drowsy time is also present. In RIGHT LATERAL RECUMBENT, a δ wave of 0.5 to 3 Hz mainly occupies, from which it is seen that the time of the second-stage sleep is long. In LEFT LATERAL RECUMBENT, a θ wave of 4 to 7 Hz mainly occupies, and in LEFT LATERAL RECUMBENT, though sleep is lighter than in RIGHT LATERAL RECUMBENT, the first-stage to second-stage sleep mainly occupies. In SITTING as well, a θ wave of 4 to 7 Hz mainly occupies, but its gain is smaller than in LEFT LATERAL RECUMBENT and other gain of a β wave of 14 Hz or higher is also higher than in the other postures. Therefore, in SITTING, the first-stage to second-stage sleep time is also relatively long, but wakefulness also appears relatively highly frequently.

In the time-series waveform of the head biological signal obtained from the head biological signal detection sensor 10, the wakefulness degree change index estimation unit 122 identifies a waveform component having an amplitude larger than preceding and succeeding amplitudes by a predetermined magnitude or more, and estimates that the waveform component is an index indicating a change in the wakefulness degree. For example, in the data in FIG. 16(b) from the experiment conducted while the subject is supine, waveform components whose amplitudes temporarily become large appear near 45 seconds, near 50 seconds, near 130 seconds, near 160 seconds, near 210 seconds, near 280 to 290 seconds, near 305 seconds, near 320 to 340 seconds, and so on. Many of these waveform components have amplitudes 1.5 times or more as large as the preceding and succeeding amplitudes. "HIGH", "LOW", and "DROWSY" expressing the states, written in the upper column in FIG. 16(b), indicate wakefulness degrees self-reported by the subject. Note that, in this sleep experiment conducted while the subject was supine, the subject was mainly in the wakefulness state as described above and thus could make the self-report.

In this time-series waveform, an instant near 48 seconds to 50 seconds when the large amplitude appears substantially coincides with an instant when the wakefulness degree changes from "HIGH" to "LOW". This is also the same with an instant near 130 seconds when the large amplitude appears. When large amplitudes intermittently appear near 280 to 290 seconds, the wakefulness degree changes from "LOW" to "DROWSY" before and after this, and thereafter, near 320 seconds to 340 seconds, the "DROWSY" state continues. In this manner, according to the magnitude of the amplitude of the waveform component in the time-series waveform in FIG. 16(b), for example, in the case where a waveform component having an amplitude 1.5 times or more as high as the amplitudes of the preceding and succeeding waveform components, this can be considered as an index indicating the wakefulness degree change.

Therefore, according to this embodiment, in the brain activity state analysis unit 120, the sleep stage identifying unit 121 is capable of estimating a sleep stage, and the wakefulness degree change index estimation unit 122 is capable of identifying an instant when the wakefulness degree changes. That is, in this embodiment, the use of the data of the head biological signal obtained from the head biological signal detection sensor 10 makes it possible to estimate a sleep stage and also estimate an instant when the wakefulness degree changes without using an electroencephalograph.

Here, FIGs. 21 to FIGs. 28 explain cases where the brain activity state analysis unit 120 applies short-time Fourier transform to the waveforms of the head biological signals to create image data (STFT plots), which are the analysis results thereof, showing time and a variation degree of a frequency power spectrum, and cases where correlograms are found.

FIGs. 21 to FIGs. 24 illustrate examples of the analysis results by the brain activity state analysis unit 120 using the data from the sleep experiment 1 conducted while the subject is supine, left lateral recumbent, right lateral recumbent, and sitting, and FIGs. 25 to FIGs. 28 illustrate examples of the analysis results by the brain activity state analysis unit 120 using the data from the sleep experiment 2 conducted while the subject is supine, left lateral recumbent, right lateral recumbent, and sitting. In FIGs. 21 to FIGs. 28, three characterizing time zones in each posture are extracted for analysis. Further, in each of FIGs. 21 to FIGs. 28, (a) illustrates waveforms of an electrocardiogram measured at the same time with the head biological signal, (b) illustrates waveforms of the head biological signal, and (c) illustrates STFT plots where the center frequency is 10 Hz, (d) illustrate STFT plots where the center frequency is 5 Hz, and (e) illustrates correlograms.

For example, the comparison between the STFT plots in the wake stage in FIGs. 21 and the first stage in FIGs. 22 show that their image features are different. The comparison between the correlograms in these drawings also shows that they are different in autocorrelation factor (ACF) value and graph shape. On the other hand, from the comparison between the wake stages of the sleep experiments 1, 2 in FIGs. 21 and FIGs. 25, common pattern and graph shape are recognized both in the STFT plots and in the collerograms. From the comparison between the first stages in FIGs. 22 and FIG. 26, a common characteristic is also recognized in at least the STFT plots. Therefore, by classifying these image data, graph shapes, and so on into patterns, it is possible to use them for the determination of a sleep stage. In the determination of a sleep stage, co-using the determination using the STFT plots and the correlograms with the aforesaid determination using the time-series waveform of the frequency analysis result enhances determination accuracy.

The heartbeat-related biological signal analysis unit 130 extracts, from the time-series waveform of the detected head biological signal, a heartbeat-related biological signal generated by cardiac motion and including at least one of an apical beat component and a heart sound component and analyzes the extracted heartbeat-related biological signal.

The present inventor has proposed, in Japanese Patent Application No. 2021-107109, a means for frequency-analyzing a biological signal obtained from the back or the thorax of a person to find a boundary frequency (BF) between apical beat-induced vibration and heart sound-induced vibration. The biological signal obtained from the back or the thorax of a person is an assembly of various biosounds and in-vivo vibrations, while the apical beat is vibration hidden behind a waveform of heart sound, and it has conventionally been difficult to separate them. However, using the boundary frequency for distinguishing the apical beat waveform and the heart sound waveform, which was found by the present inventor in the aforesaid application, makes it possible to separate them and to infer the biological states in which the apical beat and the heart sound are involved respectively, from one biological signal data.

The heartbeat-related biological signal analysis unit 130 of this embodiment frequency-analyzes the time-series waveform of the head biological signal obtained from the head biological signal detection sensor 10 to find the aforesaid boundary frequency by the method disclosed in Japanese Patent Application No. 2021-107109, thereby finding an apical beat waveform and a heart sound waveform. FIG. 29(b) illustrates an example of a time-series waveform of a head biological signal, FIG. 29(c) illustrates an apical beat waveform obtained after the boundary frequency is obtained, and FIG. 29(d) illustrates a heart sound waveform. Note that FIG. 29(a) is a waveform of an electrocardiogram measured at the same time.

FIG. 30(b) illustrates a thorax biological signal obtained when a detection sensor (sensor denoted by reference sign 1000 in FIG. 1) having the same configuration as that of the head biological signal detection sensor 10 of this embodiment is attached to the thorax, and FIGs. 30(c), (d) illustrate data of an apical beat waveform and a heart sound waveform that are obtained based on a boundary frequency found from this thorax biological signal. FIG. 31(b), similarly to FIG. 30(b), illustrates a buttocks biological signal obtained when the detection sensor is attached to the buttocks, and FIGs. 31(c), (d) illustrate data of an apical beat waveform and a heart sound waveform that are obtained based on a boundary frequency found from this buttocks biological signal. Note that FIG. 30(a) and FIG. 31(a) are waveforms of the same electrocardiogram as that in FIG. 29(a).

Further, FIGs. 32(a) to (c) are charts illustrating the frequency analysis results of the time-series waveforms of the head, thorax, and buttocks biological signals. In the drawings, "Parietal" represents the frequency analysis result of the head biological signal, "Front" represents the frequency analysis result of the thorax biological signal, and "Pelvis" represents the frequency analysis result of the buttocks biological signal. Note that FIGs. 32(a) to (c) are the results calculated using data in different time zones.

The apical beat waveform and the heart sound waveform in FIGs. 29(c), (d) found from the head biological signal are closely analogous to the apical beat waveforms and the heart sound waveforms in FIGs. 30(c), (d) and FIGs. 31(c), (d) found from the thorax biological signal and the buttocks biological signal. Further, in the three data in FIGs. 32(a) to (c), gain increases/decreases in some parts of the head biological signal and the buttocks biological signal do not necessarily agree with that in the thorax biological signal. There is a possibility that, because of the distance from the heart, the head biological signal and the buttocks biological signal are influenced by some damping, and in the case of the head, damping due to skull vibration also has an influence. Other possible influences are differences in the shape and size of the pelvis and the skull. Nevertheless, the frequency analysis results of the head biological signal, the thorax biological signal, and the buttocks biological signal in FIGs. 32(a) to (c) present the same tendency as a whole.

Therefore, detecting the head biological signal makes it possible to extract a cardiac motion-induced heartbeat-related biological signal including at least one of an apical beat component and a heart sound component.

FIG. 33 to FIG. 41 are explanatory charts of other analysis cases by the heartbeat-related biological signal analysis unit 130. They are all obtained through the analyses using partial data of the head biological signals in the sleep experiment 2 conducted while the subject is supine, sitting, left lateral recumbent, and right lateral recumbent at rest and wakefulness. FIG. 33 and FIG. 34 are charts illustrating part of time-series waveforms of electrocardiograms (ECG), finger plethysmograms (PPG), head biological signals (Parietal APW), apical beat waveforms (Parietal CAB) found from the head biological signals, and heart sound waveforms (Parietal CAS) found from the head biological signals. FIG. 35 and FIG. 36 are charts illustrating the frequency analysis results of the data in FIG. 33 and FIG. 34. FIG. 37 illustrates STFT plots of the head biological signals (Parietal APW), and FIG. 38 illustrates STFT plots of the apical beat waveforms (Parietal CAB) found from the head biological signals. FIG. 39 illustrates correlograms of the head biological signals (Parietal APW), FIG. 40 illustrates correlograms of the apical beat waveforms (Parietal CAB) found from the head biological signals, and FIG. 41 illustrates correlograms of the heart sound waveforms (Parietal CAS) found from the head biological signals.

Note that in FIG. 33 and FIG. 34 out of FIG. 33 to FIG. 41, the charts except the electrocardiograms (ECG) and the finger plethysmograms (PPG) are all charts found using the head biological signals obtained from the head biological signal detection sensor 10. Since the head biological signal is information on intracranial sound/vibration accompanying cerebral blood flow, the analytical plots obtained based on the head biological signal, such as the time-series waveforms and so on of the frequency analysis results illustrated in these drawings, the aforesaid FIGs. 6 to FIGs. 13, and so on are collectively referred to as "Phono Encephalo Graphy" in this specification. The description below will use the expression "phono-encephalo-graph" in some cases.

First, the comparison of the phono-encephalo-graphs represented by the time-series waveforms in FIG. 33 and FIG. 34 shows that a negative wave below the baseline is output with a larger magnitude in the data of the apical beat waveform in SITTING. This indicates that there is an influence of gravity in SITTING. Further, in the apical beat waveform, a harmonic component of around 10 Hz works in SUPINE, while in LEFT LATERAL RECUMBENT AND RIGHT LATERAL RECUMBENT, such a harmonic component is not seen (see the frequency analysis results in FIG. 35 and FIG. 36). The comparison of the frequency analysis results in FIG. 35 and FIG. 36 shows that the amplitude is high at around 1 Hz and a pendulum motion is seen in SITTING and RIGHT LATERAL RECUMBENT, but in SUPINE AND LEFT LATERAL RECUMBENT, an amplitude is not high at around 1 Hz. Therefore, from these phono-encephalo-graphs, it can be inferred that the left side of the heart has a tendency of serious left ventricular hypertrophy.

The comparison of the apical beat waveforms represented by the correlograms in FIG. 40 shows that, in SITTING and RIGHT LATERAL RECUMBENT, a pendulum motion of the heart is captured, but as compared with SUPINE and LEFT LATERAL RECUMBENT, a harmonic component is not superimposed, and damping is also speedily occurring. From the data in SUPINE AND LEFT LATERAL RECUMBENT, it can be read that the heart is not moving much and in particular, in SUPINE, more harmonic components are superimposed. Therefore, from these correlograms, it is also possible to infer the tendency of the left ventricular hypertrophy of the subject.

In the heart sound waveforms represented by the correlograms in FIG. 41, the shapes of phases in SITTING and RIGHT LATERAL RECUMBENT resemble those of the first and second heart sounds and thus the first sound and the second sound are captured relatively clearly, and it can also be read that damping according to blood flow is occurring after the first sound is captured. On the other hand, in SUPINE AND LEFT LATERAL RECUMBENT, the second sound is difficult to capture, and in particular, in LEFT LATERAL RECUMBENT, the influence of noise is large, and the occurrence of damping cannot be read. This is also a sign appearing when the subject takes a posture where a heavy part of the heart is on the lower side and indicates left ventricular hypertrophy.

On the other hand, in FIG. 39, the correlograms of the time waveforms of the head biological signals include the features of both the apical beat waveforms in FIG. 40 and the heart sound waveforms in FIG. 41, and the motions corresponding to blood flow and cardiac contraction are captured in SITTING and RIGHT LATERAL RECUMBENT, but only a little change is seen in LEFT LATERAL RECUMBENT. Therefore, from this FIG. 39, it is also possible to find the tendency of left ventricular hypertrophy.

In the STFT plots in FIG. 37 and FIG. 38 as well, it is seen that an appearing frequency band differs depending on the posture. In both FIG. 37 and FIG. 38, a low-frequency band is dominant in SITTING. On the other hand, in SUPINE, it can be read that a high frequency relatively often appears. The comparison between RIGHT LATERAL RECUMBENT and LEFT LATERAL RECUMBENT shows that a low-frequency band more strongly appears in RIGHT LATERAL RECUMBENT. Therefore, the use of the STFT plot also makes it possible to infer the presence of a certain heart disease.

Therefore, regarding the estimation using the time-series waveforms in FIG. 33 and FIG. 34, the estimation using the charts of the frequency analyses (FFT) in FIG. 35 and FIG. 36, the estimation using the STFT plots in FIG. 37 and FIG. 38, and the estimation using the correlograms in FIG. 39 to FIG. 41, by storing how the phono-encephalo-graphs corresponding to heart diseases such as left ventricular hypertrophy appear, as a database in a computer, the heartbeat-related biological signal analysis unit 130 is capable of performing the identification using pattern matching or AI and inferring a heart disease. Further, the heartbeat-related biological signal analysis unit 130 can also be configured to output the final estimation result after considering a plurality of kinds of estimation results instead of using only one kind of estimation out of the estimation using the time-series waveforms in FIG. 33 and FIG. 34, the estimation using the charts of the frequency analyses (FET) in FIG. 35 and FIG. 36, the estimation using the STFT plots in FIG. 37 and FIG. 38, and the estimation using the correlograms in FIG. 39 to FIG. 41.

That the heartbeat-related biological signal can be thus extracted from the head biological signal indicates that the head biological signal detection device 1 of this embodiment captures information on intracranial cerebral blood flow. Specifically, as described above, the state of the intracranial cerebral blood flow is the state of blood that has come from the heart through the internal carotid arteries and the external carotid arteries to flow in the blood vessels in the brain and reflects the supply speed of oxygen which will be energy for the brain activity. Therefore, by analyzing the head biological signal, it is possible to capture information on cerebral blood flow to estimate a sleep stage change and a wakefulness degree change which cause a variation in cerebral blood flow.

Further, as described above, an intracranial pressure (ICP) is the pressure in the space present between the brain parenchyma and the skull and filled with liquid, and when a cerebral hemorrhage or a cerebral edema occurs due to an external head injury or a brain tumor becomes large, ICP increases to cause vascular compression, resulting in a reduction in cerebral blood flow. The vascular compression reduces a damping ratio and increases vascular elasticity, generating a damped free vibration waveform having only low-frequency components with low damping. An increase in blood flow increases a damping ratio and reduces vascular elasticity, generating a damped free vibration waveform with high damping in which low-frequency and high-frequency components are mixed, that is, in which small-amplitude harmonic components with high damping are superimposed. Therefore, if abnormality such as a cerebral hemorrhage occurs, a head biological signal also presents a sign different from that in a normal state. Therefore, the brain activity state analysis unit 120 of the head biological signal analysis unit 110 can be provided with a brain disease estimation unit 123 (see FIG. 4) that analyzes a time-series waveform of the head biological signal and infers that abnormality such as a cerebral hemorrhage is occurring in the case where an amplitude variation of its waveform component is far greater than in the normal state or the frequency analysis shows that a gain of a frequency band different from that in the normal state becomes large.

### (Sleep Experiment 4)

In the above-described sleep experiments 1 to 3, as the sleep stage identifying unit 121, used is a means that frequency-analyzes the time-series waveform of the head biological signal to find the change tendency of the main frequency band, thereby identifying the sleep stage, but it is also possible to identify the sleep stage from the time-series waveform of the head biological signal.

FIG. 42 to FIG. 47 are data representing the measurement results of a sleep experiment 4 conducted on the same subject as the subject in the sleep experiments 1 to 3. In the sleep experiment 4, an electrocardiogram (ECG), respiration, a head biological signal (Parietal APW), a thorax biological signal (Front APW), and a buttocks biological signal (Pelvis APW) are simultaneously measured, and the measurement results are illustrated in FIG. 42 to FIG. 47. Further, during the experiment, blood pressure is also measured.

FIG. 42 illustrates data obtained while the subject is sitting and in the wake stage at rest, FIG. 43 illustrates data obtained while the subject is left lateral recumbent and in the first stage, FIG. 44 illustrates data obtained while the subject is right lateral recumbent and in the first stage, FIG. 45 and FIG. 46 both illustrate data obtained while the subject is right lateral recumbent and in the second stage, and FIG. 47 illustrates data obtained while the subject is left lateral recumbent and in the third stage. Here, whether the subject was in the first stage or the second stage was determined in consideration of the self-report by the subject regarding whether or not the subject could recognize sound (the first stage if the subject could recognize sound, and the second stage if the subject could not recognize sound) and a state externally observed by an observer, such as whether the subject appeared drowsy (first stage) or appeared to have passed the drowsy state to fall into deep sleep (second stage). Whether the subject was in the second stage or the third stage was determined based on whether or not the subject reacted to try to wake up from sleep when a pressure was given to his/her arm at the time of the measurement of his/her brachial blood pressure (the second stage if he/she reacted, and the third stage if he/she did not react).

From the comparison of these data, it is seen that, in the wake stage at rest in FIG. 42, the respiration stably occurs once per three to four seconds, and the waveform of the head biological signal changes with substantially constant cycle and amplitude. The waveforms of the thorax biological signal and the buttocks biological signal are also stable in both cycle and amplitude.

In the first stage in FIG. 43, the cycle of respiration is stably once per about four seconds. The head biological signal has a smaller amplitude than that in the wake stage in FIG. 42, and instants when its amplitude greatly changes are between respiration and respiration (near 457.5 to 458.5 seconds, near 461.5 to 462.5 seconds). In the data in the first stage in FIG. 44 as well, instants when the amplitude of the head biological signal becomes large deviate from respiratory instants. In the data in the second stage in FIG. 45 and FIG. 46, instants when the amplitude of the head biological signal becomes large also deviate from respiratory instants.

On the other hand, in the third stage in FIG. 47, the amplitude of the head biological signal becomes large at respiratory instants near 503 to 504 seconds, near 507 to 508 seconds, and near 511 to 512 seconds, and the respiratory instants and the amplitude increase instants substantially coincide with each other.

From the above, it follows that the sleep stage can be determined as the third stage in the case where the respiratory instants and the instants when a great amplitude change of the head biological signal appears substantially coincide with each other, and can be determined as the first stage or the second stage in the case where these instants deviate. Therefore, the sleep stage identifying unit 121 is capable of determining whether the sleep stage is the first stage/the second stage or the third stage by setting a threshold value for determining whether an amplitude change of the head biological signal is equal to a predetermined level or more and setting a threshold value of a deviation width of an instant when the amplitude change on the predetermined level or more occurs in the head biological signal from a respiratory instant.

Further, as for the thorax biological signals, the periods of their waveforms in the second stage in FIG. 45 and FIG. 46 are noticeably longer than in the wake stage at rest in FIG. 42. Regarding this point, the periods of their waveforms in the first stage in FIG. 43 and FIG. 44 are not much longer than in the wake stage in FIG. 42. Therefore, in the case where data of the thorax biological signal measured at the same time can be used, it is possible for the sleep stage identifying unit 121 to determine the distinction between the first stage and the second stage by setting a threshold value regarding by how much the period is longer than that in the thorax biological signal in the wake stage.

Incidentally, in the data of the buttocks biological signals, the amplitudes of their waveforms in the first stage, the second stage, and the third stage tend to be smaller than that of the waveform in the wake stage, and this difference is usable for the determination on whether the wake stage has ended and the first stage is entered, but there is not a conspicuous difference among the data in the first stage, the second stage, and the third stage. Therefore, in the case where the sleep stage identifying unit 121 identifies a sleep stage based on the relation with the respiratory period, the use of the data of the head biological signal is preferable, and co-using data of the thorax biological signal with it makes it possible to more accurately identify the sleep stage.

As the sleep stage identifying unit 121, it is of course possible to co-use the means for identifying the sleep stage from the time-series waveform of the head biological signal (more preferably, the means for identifying the sleep stage by co-using the time-series waveform of the thorax biological signal) and the means, which is described in the aforesaid sleep experiments 1 to 3, for finding a change tendency of the main frequency band by frequency-analyzing the time-series waveform of the head biological signal, to identify the sleep stage, and this can enhance the accuracy in the identification of the sleep stage.

Note that the above-described biological state estimation device 100 is constituted by a computer (also including a personal computer, a microcomputer assembled in a device, and the like) that processes the head biological signal obtained by the head biological signal detection device 1 to estimate the sleep stage or the like as described above. Then, a computer program for the execution of procedures caused to function as the brain activity state analysis unit 120 and the heartbeat-related biological signal analysis unit 120 which form the biological signal analysis unit 110, and the sleep stage identifying unit 121, the wakefulness degree change index estimation unit 122, and the brain disease estimation unit 123 which form the brain activity state analysis unit 120 is stored in a storage unit (including not only a recording medium such as a hard disk built in the computer (biological state estimation device 100) but also any of various removable recording mediums and a recording medium of another computer connected through communication means). Note that the biological state estimation device 100 can be implemented using an electronic circuit having one storage circuit or more in which the computer program implementing the brain activity state analysis unit 120 and the heartbeat-related biological signal analysis unit 120 which form the biological signal analysis unit 110, and the sleep stage identifying unit 121, the wakefulness degree change index estimation unit 122, and the brain disease estimation unit 123 which form the brain activity state analysis unit 120 is incorporated.

Further, the computer program can be provided by being stored in a recording medium. The recording medium storing the computer program may be non-transitory. The non-transitory recording medium is not limited and its examples include recording mediums such as a flexible disk, a hard disk, CD-ROM, MO (magneto-optical disk), DVD-ROM, and a memory card. The computer program can also be installed in the computer by being transferred to the computer through communication lines.

### Explanation of Reference Signs

- 1: head biological signal detection device
- 10: head biological signal detection sensor
- 11: three-dimensional knitted fabric
- 12: housing film
- 13: microphone
- 14: case
- 15: gel
- 20: sensor support member (cap-type sensor support member)
- 21: crown
- 23: fastening structure
- 100: biological state estimation device
- 110: head biological signal analysis unit
- 120: brain activity state analysis unit
- 121: sleep stage identifying unit
- 122: wakefulness degree change index estimation unit
- 123: brain disease estimation unit
- 130: heartbeat-related biological signal analysis unit

## Claims

1. A head biological signal detection device comprising:
a head biological signal detection sensor that includes: a three-dimensional knitted fabric having a pair of ground knitted fabrics facing each other at an interval and a connecting yarn knitted between the pair of ground knitted fabrics to keep the interval between the pair of facing ground knitted fabrics; a housing film covering the three-dimensional knitted fabric; and a microphone that detects sound/vibration propagated through the three-dimensional knitted fabric, the head biological signal detection sensor being disposed with an abutting surface thereof in contact with a surface of a head, to detect a head biological signal which is sound/vibration generated by cerebral blood flow; and
a sensor support member with which the head biological signal detection sensor is supported on the head in a manner that the abutting surface is in contact with the surface of the head and the connecting yarn sag to make the interval between the pair of facing ground knitted fabrics narrower than that under no load,
wherein microvibration occurring in the three-dimensional knitted fabric or the housing film due to movement of the head while the biological signal detection device is worn works as noise to generate a stochastic resonance phenomenon between the noise and the head biological signal, and the head biological signal is detected by the microphone.

2. The head biological signal detection device according to claim 1, wherein the sensor support member has a worn state maintaining structure that, when the head biological signal detection sensor is supported on the head, causes the connecting yarn to sag such that the interval between the pair of facing ground knitted fabrics becomes a 40 to 95% interval of a reference interval under no load.

3. The head biological signal detection device according to claim 2, wherein the sensor support member is of a cap type, on an inner peripheral surface of a crown of the sensor support member, the head biological signal detection sensor is attached, and on a lower edge of the crown, a fastening structure as the worn state maintaining structure is provided to maintain a position of the head biological signal detection sensor by being in close contact with a periphery of the head.

4. The head biological signal detection device according to claim 3, wherein the fastening structure includes at least one of an elastic member exhibiting an elastic force in a direction of the periphery of the head and a belt-shaped member whose length is adjustable.

5. The head biological signal detection device according to claim 3 or 4, wherein the crown is formed of a three-dimensional knitted fabric.

6. The head biological signal detection device according to any one of claims 1 to 5,
wherein the microphone of the head biological signal detection sensor is attached to an outer side of the housing film,
wherein the head biological signal detection sensor has a synthetic resin case covering the microphone and an external disturbance mixture preventing member functioning in the case to prevent an external disturbance from mixing to the microphone, and
wherein a surface, of the housing film, opposite to the surface to which the microphone is attached is the abutting surface.

7. The head biological signal detection device according to claim 6, wherein the external disturbance mixture preventing member is a gel.

8. A biological state estimation device comprising a head biological signal analysis unit that receives and analyzes the head biological signal obtained from the head biological signal detection device according to any one of claims 1 to 7, estimates a predetermined state of a person together with an occurrence instant of the state, and outputs a result of the estimation.

9. The biological state estimation device according to claim 8, wherein the head biological signal analysis unit includes a brain activity state analysis unit that outputs an index regarding an activity state of a brain, together with an occurrence instant of the index.

10. The biological state estimation device according to claim 9, wherein the brain activity state analysis unit includes a sleep stage identifying unit that frequency-analyzes the head biological signal obtained from the head biological signal detection device to find, in time series, a main frequency band of the head biological signal, and based on the main frequency band, identifies a sleep stage out of sleep stages including a wake stage, a drowsiness stage to a deep sleep stage, and a REM sleep stage.

11. The biological state estimation device according to claim 9 or 10, wherein the brain activity state analysis unit includes a wakefulness degree change index estimation unit that, in a time-series waveform of the head biological signal obtained from the head biological signal detection device, identifies a waveform component whose amplitude is larger than preceding and succeeding amplitudes by a predetermined magnitude or more, and estimates that the waveform component is an index indicating a change in a wakefulness degree.

12. The biological state estimation device according to any one of claims 8 to 11, wherein the head biological signal analysis unit includes a heartbeat-related biological signal analysis unit that extracts, from the time-series waveform of the head biological signal obtained from the head biological signal detection device, a heartbeat-related biological signal generated by cardiac motion and containing at least one of an apical beat component and a heart sound component, and analyzes the heartbeat-related biological signal.

13. The biological state estimation device according to claim 10, wherein the sleep stage identifying unit compares an instant when the amplitude of the time-series waveform obtained from the head biological signal detection device becomes large with a respiratory period, and identifies the sleep stage based on whether or not the instant coincides with the respiratory period.
